(19) Europäisches Patentamt
European Patent Office
Office européen des brevets

(11) **EP 2 771 408 B1**

(12) **EUROPÄISCHE PATENTSCHRIFT**

(45) Veröffentlichungstag und Bekanntmachung des Hinweises auf die Patenterteilung:
**25.05.2016 Patentblatt 2016/21**

(51) Int Cl.:
*C09C 1/00* (2006.01)   *C09D 5/36* (2006.01)
*A61Q 3/02* (2006.01)   *A61K 8/81* (2006.01)

(21) Anmeldenummer: **13709893.5**

(22) Anmeldetag: **13.03.2013**

(86) Internationale Anmeldenummer:
**PCT/EP2013/055166**

(87) Internationale Veröffentlichungsnummer:
**WO 2013/135782 (19.09.2013 Gazette 2013/38)**

(54) **KOMPOSITPARTIKEL, VERFAHREN ZU DEREN HERSTELLUNG UND VERWENDUNG DERSELBEN**

COMPOSITE PARTICLES, METHOD FOR PRODUCTION THEREOF, AND USE THEREOF

PARTICULES COMPOSITES, PROCÉDÉ DE LEUR PRÉPARATION ET LEUR UTILISATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.03.2012 DE 102012102165**

(43) Veröffentlichungstag der Anmeldung:
**03.09.2014 Patentblatt 2014/36**

(73) Patentinhaber: **Eckart GmbH**
**91235 Hartenstein (DE)**

(72) Erfinder:
• **DRESSEL, Bernd**
**91235 Velden (DE)**
• **ALBERT, Roland**
**91238 Offenhausen (DE)**
• **BUDAKOV, Oleg**
**Moskau 119333 (RU)**

(74) Vertreter: **Louis Pöhlau Lohrentz**
**Patentanwälte**
**Postfach 30 55**
**90014 Nürnberg (DE)**

(56) Entgegenhaltungen:
EP-A1- 1 520 876   DE-A1- 10 313 663
DE-A1- 19 941 607   DE-A1-102008 064 201
US-A1- 2011 112 234

**Beschreibung**

Die vorliegende Erfindung betrifft Kompositpartikel, umfassend ein Trägermaterial sowie wenigstens ein Perlglanzpigment, ein Verfahren zu deren Herstellung sowie deren Verwendung.

Perlglanzpigmente sind in trockener pulvriger Form extrem staubend. Um die Handhabung von Perlglanzpigmenten, beispielsweise beim Dosieren, zu vereinfachen, werden Perlglanzpigmente in mit Lösemittel angefeuchteter oder pastöser Form angeboten. Nachteilig ist, dass bei diesen Darreichungsformen Lösemittel in die jeweilige Applikation, beispielsweise in Farben, Lacke, Kosmetika, etc., eingetragen werden kann.

Als Alternative werden auch Perlglanzpigmentgranulate angeboten, bei denen die Perlglanzpigmente mit Harzen, Bindemitteln, etc. in gebundener, mithin nichtstaubender Form, vorliegen. Nachteilig bei dieser Variante ist, dass sich teilweise Agglomerate von Perlglanzpigmentgranulaten bilden, die in der Applikation nicht vollständig zerfallen. Auch kann es aufgrund der verwendeten Harze, Bindemittel, etc. zu Inkompatibilitäten mit dem jeweiligen Applikationsmedium kommen.

Die DE 10 2008 064 201 A1 betrifft nichtstaubende Pigmentgranulate, welche auf einem Träger basieren und mittels eines Haftvermittlers mit Effektpigmenten beschichtet sind. Als Haftvermittler kommen wässrige Emulsionen, Emulsionen und Dispersionen auf der Basis von acrylierten Polypropylenen und niedrigchlorierten Polypropylenen oder Wachsemulsionen zum Einsatz. Das Trägermaterial wird mit dem Effektpigment durch die Emulsion partiell oder vollständig beschichtet oder umhüllt. Gemäß der Lehre der DE 10 2008 064 201 A1 sind die mechanischen Eigenschaften von mittels wässrigen Haftvermittlern mit plättchenförmigen Effektpigmenten beschichteten kunststoffbasierenden Trägern, ohne Vorbehandlung, wie z.B. Corona-Entladung oder Beflammung, oftmals mangelhaft. Dies äußert sich insbesondere in Form von staubigem Abrieb und Haftungsverlust der Beschichtung vom Trägersubstrat in Form einer Delaminierung. Nachteilig bei dieser Variante ist auch, dass die verwendeten Haftvermittler zu Inkompatibilitäten bei der Applikation führen können.

EP 1 520 876 A1 betrifft eine Pigmentpräparation für Kunststoffe. DE 199 41 607 A1 betrifft eine Pigmentmischung, die BiOCI-Pigmente enthält. DE 103 13 663 A1 betrifft eine Bindemittel-haltige hochglänzende, nichttoxische Perglanzpigmentpräparation. US 2011/0112234 A1 betrifft eine Pigmentmischung. DE 10 2008 064 201 A1 betrifft Pigmentgranulate.

Aufgabe der vorliegenden Erfindung ist es, Perlglanzpigmente in einer wenig oder nicht staubenden Form bereitzustellen. Insbesondere sollen die Perlglanzpigmente dabei kompatibel mit den unterschiedlichsten Applikationsmedien sein. Weiterhin soll diese Darreichungsform der Perlglanzpigmente auf einfache Art und Weise zugänglich sein.

Die der vorliegenden Erfindung zugrunde liegende Aufgabe wird durch Bereitstellung von Kompositpartikeln, welche ein Trägermaterial und wenigstens ein Perlglanzpigment umfassen, gelöst, wobei das Trägermaterial und das wenigstens eine Perlglanzpigment ohne Haftmittel aneinander gebunden vorliegen, wobei das Perlglanzpigment auf der Oberfläche des Trägermaterials und von dem Trägermaterial nicht umhüllt oder eingekapselt ist.

Im Sinne der Erfindung wird unter dem Merkmal "ohne Haftmittel" verstanden, dass die Perlglanzpigmente nicht über ein separat aufgebrachtes oder angeordnetes Haftmittel oder einen separat aufgebrachten oder angeordneten Haftvermittler an das Trägermaterial gebunden sind. Die Notwendigkeit eines Haftvermittlers wird beispielsweise in DE 10 2008 064 201 A1 beschrieben.

Bevorzugte Weiterbildungen sind in den abhängigen Unteransprüchen 2 bis 10 angegeben.

Weiterhin wird die der Erfindung zugrunde liegende Aufgabe durch Bereitstellung eines Verfahrens zur Herstellung der erfindungsgemäßen Kompositpartikel gelöst, wobei das Verfahren folgenden Schritt umfasst:

Mischen von Trägermaterial und wenigstens einem Perlglanzpigment, wobei das Trägermaterial eine wenigstens teilweise erweichte Oberfläche aufweist.

Des Weiteren ist Gegenstand der Erfindung die Verwendung der erfindungsgemäßen Kompositpartikel zur Beschichtung von Substraten, vorzugsweise Tapeten oder Druckerzeugnissen, sowie in einem Beschichtungsmaterial oder als Beschichtungsmaterial. Bei dem Beschichtungsmaterial kann es sich um Farben, Druckfarben, Lacke und Kosmetika handeln. Bei dem Kosmetikum kann es sich um Nagellacke, Cremes, Lotionen, Puder, etc. handeln. Vorzugsweise ist das Kosmetikum ein Nagellack.

Insbesondere eignen sich die erfindungsgemäßen Kompositpartikel zur optischen Veredlung von Substraten, insbesondere Tapeten, Druckerzeugnissen, etc.. Dabei können die Substrate, insbesondere Tapeten, Druckerzeugnisse, etc., bereits bedruckt oder mit Mustern versehen sein.

Die Erfinder haben überraschend festgestellt, dass Perlglanzpigmente in trockener und zugleich wenig oder nicht staubender Form bereitgestellt werden können, wenn das Trägermaterial und die Perlglanzpigmente ohne Haftmittel aneinander gebunden vorliegen.

Dabei können an einem Trägermaterial ein einziges Perlglanzpigment oder mehrere Perlglanzpigmente gebunden vorliegen. Das Trägermaterial ist vorzugsweise partikulär ausgebildet. Vorzugsweise sind an einem Trägerpartikel mehrere Perlglanzpigmente gebunden. Vorzugsweise ist das wenigstens eine Perlglanzpigment bzw. sind die Perlglanzpigmente in weitgehend flächigem Kontakt mit dem Trägermaterial verbunden. Auf dem Trägermaterial, vorzugsweise Trägerpartikel, können in Abhängigkeit von der Größe des Trägerpartikels eine Vielzahl von Perlglanzpigmentpartikeln, beispielsweise bis zu 100, bis zu 250 oder bis zu 500 Perlglanzpigmente, aufgebracht und gebunden sein.

Der Anteil an Perlglanzpigment bezogen auf Gesamtgewicht der Kompositpartikel liegt bevorzugt in einem Bereich von 0,1 bis 20 Gew.-%, besonders bevorzugt in einem Bereich von 0,2 von 15 Gew.-%, weiter besonders bevorzugt in einem Bereich 0,3 bis 12 Gew.-% und ganz besonders bevorzugt in einem Bereich von 0,5 bis 8 Gew.-%, jeweils bezogen auf das Gesamtgewicht der Kompositpartikel.

Erfindungsgemäß liegt das Perlglanzpigment auf der Oberfläche des Trägermaterials und wird von diesem nicht umhüllt oder nicht eingekapselt. Insofern unterscheidet sich das erfindungsgemäße Kompositpartikel grundlegend von einer Pigmentpräparation zur Herstellung von Masterbatches.

Durch die Bindung der Perlglanzpigmente an das Trägermaterial, vorzugsweise Trägerpartikel, können die Perlglanzpigmente staubarm, vorzugsweise staubfrei, gehandhabt werden. Durch die Trägerpartikel weisen die Kompositpartikel ein so hohes Gewicht auf, dass es bei der üblichen Handhabung von Perlglanzpigmenten, beispielsweise bei der Dosierung, zu keiner wesentlichen, vorzugsweise zu keiner, Staubentwicklung kommt. Des Weiteren kommt es bei den erfindungsgemäßen Kompositpartikeln vorzugsweise zu keinen Agglomerationen. Mithin sind die erfindungsgemäßen Kompositpartikel vorzugsweise agglomeratfrei.

Äußerst vorteilhaft sind die erfindungsgemäßen Kompositpartikel staubarm, vorzugsweise staubfrei, rieselbar und/oder streubar. So können die erfindungsgemäßen Kompositpartikel äußerst vorteilhaft beispielsweise auf ein mit Haft- und/oder Bindemittel versehenes Substrat, beispielsweise eine Tapete oder ein Druckerzeugnis, aufgestreut oder durch Rieselung aufgebracht werden, ohne dass es zu einer Belastung von Mensch und Umwelt durch eine merkliche Staubentwicklung kommt. Mithin bieten die erfindungsgemäßen Kompositpartikel erhebliche anwendungstechnische Vorteile. Auch sind staubarme bzw. staubfreie und dennoch rieselfähige Kompositpartikel bei der Einarbeitung in eine Druckfarbe von Vorteil.

Gemäß einer bevorzugten Variante der Erfindung sind die Perlglanzpigmente an wenigstens einer Oberfläche des Trägermaterials, vorzugsweise der Trägerpartikel, stoffschlüssig gebunden. Die stoffschlüssige Bindung wird vorzugsweise durch ein wenigstens teilweises Erweichen der Oberfläche des Trägermaterials, vorzugsweise der Trägerpartikel, und nachfolgendes Anhaften der Perlglanzpigmente an den erweichten Oberflächenbereich bewirkt. Bei dem vorzugsweise nur teilweise oberflächlichen Erweichen des Trägermaterials wird die Oberfläche klebrig, so dass die Perlglanzpigmente daran haften bleiben. Nach erfolgter Anhaftung der Perlglanzpigmente werden die so erhaltenen Kompositpartikel abgekühlt, so dass die Oberfläche des Trägermaterials, vorzugsweise der Trägerpartikel erstarrt und sich verfestigt. Mithin werden die Perlglanzpigmente ohne separates Haftmittel an das Trägermaterial, vorzugsweise die Trägerpartikel, gebunden. Die Bindung der Perlglanzpigmente wird mithin durch das Trägermaterial bewirkt.

Das Erweichen der Oberfläche des Trägermaterials, vorzugsweise der Trägerpartikel, wird vorzugsweise durch Wärmebeaufschlagung bzw. Temperaturerhöhung bewirkt. Das Erstarren und Verfestigen der erweichten Oberfläche des Trägermaterials, vorzugsweise der Trägerpartikel, erfolgt durch Abkühlen bzw. Absenken der Temperatur. Beim Erwärmen des Trägermaterials, vorzugsweise der Trägerpartikel, kann in Abhängigkeit von der gewählten Temperatur und der Zusammensetzung des Trägermaterials nicht nur die Oberfläche, sondern das komplette Trägermaterial erweichen. Entsprechendes gilt selbstverständlich auch für den nachfolgenden Abkühlvorgang.

Selbstverständlich kann die Oberfläche des Trägermaterials, vorzugsweise der Trägerpartikel, auch durch Behandeln mit einem Lösemittel angelöst und/oder erweicht werden, wonach eine Bindung der Perlglanzpigmente, vorzugsweise unter Trocknung bzw. Verflüchtigung des Lösemittels, ermöglicht wird.

Erfindungsgemäß ist es bevorzugt, eine Erweichung der Oberfläche des Trägermaterials, bevorzugt der Trägerpartikel, durch Wärmebeaufschlagung zu bewirken.

Die Bindung der Perlglanzpigmente erfolgt mithin vorzugsweise ausschließlich durch das erweichte und nachfolgend erstarrte oder verfestigte Trägermaterial bzw. das Material der Trägerpartikel.

Selbstverständlich kann es sich bei Kompositpartikeln gemäß der vorliegenden Erfindung auch um klassierte Kompositpartikel handeln, die beispielsweise durch Siebung oder mittels Zyklon erhalten werden können.

Gemäß einer bevorzugten Variante der Erfindung ist das Trägermaterial, das vorzugsweise in Form von Trägerpartikeln vorliegt, plättchenförmig. Unter plättchenförmig wird verstanden, dass das Trägermaterial, vorzugsweise die Trägerpartikel, flächig ist und eine nahezu einheitliche, vorzugsweise einheitliche, Dicke aufweist.

Gemäß einer weiteren Variante der Erfindung sind die Trägermaterialien, vorzugsweise Trägerpartikel, linsenförmig, sphärisch oder irregulär geformt.

Bei einer bevorzugten Ausführungsform liegen die Trägermaterialien plättchenförmig und polygonal vor. Weiter bevorzugt liegen die Trägermaterialien tetragonal, pentagonal, hexagonal, heptagonal, octogonal, nonagonal und/oder decagonal vor. Als sehr geeignete Polygone haben sich Tetragone, Hexagone und/oder Octogone erwiesen.

Gemäß einer weiteren bevorzugten Ausführungsform weist das plättchenförmige Trägermaterial, vorzugsweise die Trägerpartikel, eine einheitliche Formgebung, beispielsweise eine tetragonale oder hexagonale oder octogonale Form auf.

Tetragonale plättchenförmige Trägermaterialien, vorzugsweise Trägerpartikel, sind Rechtecke, beispielsweise Quadrate, Rhomben, Parallelogramme oder Trapeze.

Gemäß einer weiteren bevorzugten Weiterbildung der Erfindung weist das plättchenförmige Trägermaterial, vorzugsweise Trägerpartikel, eine hexagonale Form auf.

Gemäß einer weiteren bevorzugten Ausführungsform liegt das plättchenförmige Trägermaterial, vorzugsweise Trägerpartikel, als Kreisscheibe oder als Plättchen mit kreisförmigem Umfang vor.

Bei einer weiteren Ausführungsform liegen die Trägermaterialien in annähernd sphärischer Form oder linsenförmig vor.

Bei einer weiteren Ausführungsform liegen die Trägermaterialien in annähernd sphärischer Form vor.

Bei einer weiteren Ausführungsform liegen die Trägermaterialien annähernd linsenförmig vor.

Gemäß einer weiteren Variante der Erfindung ist das Trägermaterial, vorzugsweise Trägerpartikel, ein Kunststoff. Weiterhin ist bevorzugt, dass der Kunststoff ein thermoplastischer Kunststoff oder ein thermoplastisches Elastomer ist.

Bei dem Kunststoff kann es sich um einen sortenreinen Kunststoff oder um ein Gemisch verschiedener Kunststoffe, beispielsweise verschiedener Polymere, handeln.

Mithin enthält gemäß einer weiteren Variante der Erfindung der Kunststoff, aus dem das Trägermaterial, vorzugsweise die Trägerpartikel, hergestellt sind, lediglich einen Anteil an thermoplastischen Kunststoff oder thermoplastischem Elastomer.

Es ist von Vorteil, wenn der als Trägermaterial, vorzugsweise Trägerpartikel, verwendete Kunststoff thermoplastischen Kunststoff oder thermoplastisches Elastomer enthält oder vorzugsweise daraus besteht, da dessen Oberfläche durch Wärmebeaufschlagung erweicht bzw. angeschmolzen werden kann, so dass die Perlglanzpigmente an die erweichte bzw. teilweise geschmolzene Oberfläche binden können.

Als geeignete Kunststoffe oder Polymere können beispielsweise Polyethylen (PE), Polypropylen (PP), Polybutylen (PB), Polyisobutylen (PI), Polystyrol (PS), Poly(meth)acrylate, wie z.B. Polymethylmethacrylat (PMMA), Polyester, wie z.B. Polyethylenterephthalat (PET) und Polybutylentherephthalat (PBT), Polycarbonat (PC), Polyvinylacetat-Copolymer (PVAC), Polyvinylchlorid (PVC), Ethylenacrylsäure-Copolymer (EAA), Polyvinylacetale, wie z.B. Polyvinylbutyrale, Polyvinylbutyral (PVB), Polyvinylalkohol (PVAL), Polyolefine (PO), Polyamid (PA), Celluloseacetat (CA), Celluloseacetobutyrat (CAB), Cellulosenitrat (CN), Cellulosetriacetat (CTA), Ethylenvinylacetat (EVA), Ethylenvinylacetat-Copolymer, biologisch abbaubare Polyester, wie z. B. Polymilchsäure (Polylactit, PLA), Polyether, wie z. B. Polyethylenglykol, Polyacrylnitril oder deren Mischungen eingesetzt werden.

Bevorzugt werden als Trägermaterialien, vorzugsweise Trägerpartikel, Polyester, wie z. B. Polyethylenterephthalat (PET) und Polybutylentherephthalat (PBT) sowie Polyvinylacetale, wie z. B. Polyvinylbutyrale, Polyvinylbutyral (PVB) oder deren Mischungen eingesetzt.

Besonders bevorzugt werden als Trägermaterialien, vorzugsweise Trägerpartikel, Polyvinylbutyral (PVB) und Polyethylenterephthalat (PET) eingesetzt. Ganz besonders bevorzugt wird Polyethylenterephthalat (PET) als Trägermaterial, vorzugsweise Trägerpartikel, verwendet.

Gemäß einer bevorzugten Variante der Erfindung erweichen die Kunststoffe in einem Temperaturbereich von 40 bis 230° C, weiter bevorzugt von 45° C bis 190° C, noch weiter bevorzugt von 50 bis 170° C. Die Erweichungstemperatur kann auch als Glasübergangstemperatur Tg bezeichnet werden. Die Glasübergangstemperatur wird mit dynamischer Differenzkalorimetrie (DSC) ermittelt.

Trägermaterialien in hexagonaler Form und bestehend aus Polyethylenterephthalat (PET) sind beispielsweise von der Firma RJA Plastics GmbH, 07989 Teichwolframsdorf, Deutschland, unter den Handelsnamen Crystal Clear 0.008" 45.008E, Crystal Clear 0.016" 45.016E, Crystal Clear 0.025" 45.025E erhältlich. Sphärische oder linsenförmige Trägerpartikel aus Kunststoff können hergestellt werden, indem das oder die Polymere zunächst geschmolzen und durch Granulation, wie z. B. Unterwassergranulation, auf die gewünschte Partikelgröße und Form mittels einer Lochscheibe gebracht werden.

Bei einer weiteren Ausführungsform können die Trägermaterialien, vorzugsweise Trägerpartikel, als ungleichmäßig geformte Polymere vorliegen. Hierzu können die Polymere als Polymergranulate zunächst durch Vermahlung, z. B. in einer Stiftmühle, Kugelmühle oder Rührwerkskugelmühle, auf die gewünschte Partikelgröße gebracht werden.

Die Trägermaterialien, vorzugsweise die Trägerpartikel, sind vorzugsweise transparent und uneingefärbt. Die durch die Trägermaterialien durchgelassene bzw. transmittierte Lichtmenge besteht aus einer gerichteten und einer diffusen Komponente. Wird das Licht gleichmäßig in alle Richtungen gestreut, bewirkt dies eine Verminderung des Kontrastes und ein milchig-trübes Erscheinungsbild. Der sogenannte Haze (Trübung) wird definiert als die Lichtmenge in Prozent, die im Mittel um mehr als 2,5° vom einfallenden Lichtstrahl abweicht (Byk-Gardner, Qualitätskontrolle für Lacke und Kunststoffe, 2011/2012, S. 61/62). Vorzugsweise weichen bei den erfindungsgemäß einzusetzenden Trägermaterialien weniger als 15 %, weiter bevorzugt weniger als 10 %, noch weiter bevorzugt weniger als 5 % des einfallenden Lichts mehr als 2,5° vom einfallenden Lichtstrahl ab.

Zur Erzielung spezieller Farbeffekte können daneben auch eingefärbte, einseitig bedruckte oder einseitig beschichtete Trägermaterialien eingesetzt werden.

Zur Herstellung gefärbter plättchenförmiger Trägermaterialien, vorzugsweise Trägerpartikel, kann eine Folie, vorzugsweise vor dem Zuschneiden der entsprechenden Folie, mit einem eingefärbten handelsüblichen Bindemittel für lösemittelbasierende Tief- und Flexodruckfarben bedruckt werden. Als eingefärbte Bindemittel können hierbei Polyester, Polyamide, PVC-Copolymerisate, aliphatische und/oder aromatische Ketonharze, Melamin-Harnstoff-Harze, Melamin-FormaldehydHarze, Maleinate, Kolophoniumderivate, Polyvinylbutyrale, Casein bzw. Casein-Derivate, Ethylcellulose, Nitrocellulose und/oder aromatische und/oder aliphatische Polyurethane oder Mischungen davon, welche mit einem Farbmittel versetzt sind, eingesetzt werden. Bevorzugt werden als eingefärbtes Bindemittel Polyvinylbutyral oder Polyurethan, welche mit bis zu 20 Gew.-% Farbmittel, bezogen auf das Gesamtgewicht des Bindemittels, versetzt sind, eingesetzt.

Bei einer bevorzugten Ausführungsform werden gefärbte plättchenförmige Trägermaterialien, vorzugsweise plättchenförmige hexagonale Trägerpartikel, hergestellt, indem beispielsweise eine Polyethylenterephthalatfolie, vorzugsweise vor dem Zuschneiden der Polyethylenterephthalatfolie, mit einem eingefärbten 25 Gew.-%igen Polyvinylbutyral-Bindemittel im Tiefdruck bedruckt wird.

Selbstverständlich ist es auch möglich, bei der Herstellung plättchenförmiger Trägermaterialien, vorzugsweise Trägerpartikel, beispielsweise hexagonaler Trägerpartikel, aus einer Folie eine bereits farbige oder eingefärbte Folie zu verwenden.

Bei einer bevorzugten Ausführungsform besteht das plättchenförmige Trägermaterial, vorzugsweise Trägerpartikel nur aus einer Polymerschicht, welche optional mit einem Oberflächenadditiv beschichtet sein kann.

Die beiden Oberflächen des plättchenförmigen Trägermaterials, vorzugsweise Trägerpartikels, können aus unterschiedlichen Materialien bestehen. Das Trägermaterial, vorzugsweise Trägerpartikel, wird bei dieser Variante vorzugsweise aus Verbundfolien hergestellt, die auf herkömmliche Art und Weise, beispielsweise durch Koextrusion, Kaschierung oder Extrusionsbeschichtung, hergestellt werden können.

Alternativ kann auch eine Seite des Trägermaterials, vorzugsweise in Form einer Folie, mit einem Beschichtungsmittel oder Oberflächenadditiv versehen sein.

Erfindungsgemäß kann dadurch bewirkt werden, dass die Perlglanzpigmente nur an einer Fläche des Trägermaterials, vorzugsweise Trägerpartikel, binden. Beispielsweise kann das Beschichtungsmittel oder Oberflächenadditiv eine Haftung der Perlglanzpigmente an die beschichtete Fläche verhindern. Das Beschichtungsmittel oder das Oberflächenadditiv können beispielsweise auf eine Folienfläche aufgebracht sein, bevor aus der Folie die Trägerpartikel, beispielsweise durch Schneiden, Stanzen, Pressen, etc., hergestellt werden.

Ebenso können Verbundfolien, die zur Herstellung des Trägermaterials, vorzugsweise in Form von Trägerpartikeln, verwendet werden, eine Folienfläche aufweisen, an die Perlglanzpigmente nicht gebunden werden können.

Die selektive Bindung der Perlglanzpigmente kann dadurch bewirkt werden, dass die Erweichungstemperatur der einen Fläche des Trägermaterials, vorzugsweise der Trägerpartikel, sich von der Erweichungstemperatur der anderen Fläche unterscheidet, mithin bei einer angelegten Temperatur nur eine Fläche oberflächlich erweicht und mithin nur die erweichte Fläche eine Bindung von Perlglanzpigmenten erlaubt. Sofern die Bindung durch Anlösen des Kunststoffs unter Verwendung von Lösemittel erfolgt, kann eine selektive Bindung an eine Kunststofffläche über das unterschiedliche Löseverhalten des in der jeweiligen Schicht verwendeten Kunststoffs eingestellt werden.

Ein großer Vorteil bei der selektiven Bindung von Perlglanzpigmenten an nur eine Fläche des Trägermaterials, vorzugsweise Trägerpartikel, ist, dass der Verbrauch von Perlglanzpigmenten halbiert wird.

Selbstverständlich ist gemäß einer weiteren Ausführungsform der Erfindung auch eine beidseitige Belegung des Trägermaterials mit Perlglanzpigment möglich. Eine beidseitige Belegung kann erwünscht sein, wenn ein sehr intensiver Perlglanzeffekt erwünscht ist.

Vorzugsweise ist das Trägermaterial bzw. sind die Trägerpartikel im Wesentlichen transparent, vorzugsweise transparent.

Bei Aufbringung der erfindungsgemäßen Kompositpartikel auf ein Substrat, beispielsweise Tapete, Druckerzeugnis oder Fingernagel, ist bei einem transparenten Trägermaterial die Orientierung der Kompositpartikel nahezu ohne Bedeutung. Die optische Wirkung der Perlglanzpigmente wird dabei durch das Trägermaterial nur geringfügig beeinflusst.

Plättchenförmige Trägermaterialien in polygonaler Form oder kreisrunder Form, vorzugsweise hexagonaler oder rechteckiger Form, können durch Schneiden, Stanzen, Pressen, etc. von Folien oder Filmen aus Kunststoff hergestellt werden. Hierdurch kann eine einheitliche Partikelgröße gewährleistet werden. Herstellungsbedingt kann auch ein geringer Anteil von andersförmigen oder mehrfach geschnittenen Teilchen im Trägermaterial enthalten sein. Dieser Anteil beträgt vorzugsweise jedoch nicht mehr als 15 Gew.-%, weiter bevorzugt nicht mehr als 10 Gew.-%, weiter bevorzugt nicht mehr als 8 Gew.-%, noch weiter bevorzugt nicht mehr als 5 Gew-.%, noch weiter bevorzugt nicht mehr als 3 Gew.-%, jeweils bezogen auf das Gesamtgewicht an Trägermaterial, um das optische Erscheinungsbild der Kompositpartikel in einer Applikation nicht zu beeinträchtigen.

Gemäß einer bevorzugten Weiterbildung der Erfindung werden plättchenförmige transparente Trägermaterialien eingesetzt, welche eine polygonale Form, beispielsweise hexagonale oder rechteckige Form, oder eine kreisrunde Form aufweisen, wobei die Trägermaterialien einseitig mit einem Beschichtungsmittel oder einem Oberflächenadditiv versehen sein können, so dass die Perlglanzpigmente nicht an die beschichtete Oberfläche binden.

Bei einer weiteren Ausführungsform können auch unterschiedlich geformte Trägermaterialien, vorzugsweise Trägerpartikel, gleicher oder ähnlicher Erweichungstemperatur eingesetzt werden. Die Perlglanzpigmente können somit gleichzeitig beispielsweise auf Trägermaterialien in plättchenförmiger hexagonaler Form und Trägermaterialien in annähernd sphärischer Form aufgebracht werden. Die optischen Effekte, die mit Kompositpartikeln, welche auf einem plättchenförmigen hexagonalen Trägermaterial basieren, unterscheiden sich in der Regel von den optischen Effekten, welche mit Kompositpartikeln basierend auf einem annähernd sphärischen Trägermaterial erzielt werden können. Bei auf einem annähernd sphärischen Trägermaterial basierenden Kompositpartikel sind einem Betrachter auch außerhalb der direkten Draufsicht mehr Perlglanzpigmente zugewandt als dies bei Kompositpartikeln basierend auf einem plättchenförmigen hexagonalen Trägermaterial der Fall wäre. Dafür erscheinen auf einem annähernd sphärischen Trägermaterial basierende Kompositpartikel in der Draufsicht weniger hochglänzend als Kompositpartikel, die ein plättchenförmiges hexagonales Trägermaterial aufweisen. Allerdings ist der Glanz von Kompositpartikeln, welche auf einem annähernd sphärischen Trägermaterial basieren, deutlich weniger vom Betrachtungswinkel abhängig als der Glanz von Kompositpartikeln, welche auf einem plättchenförmigen hexagonalen Trägermaterial basieren. Selbstverständlich müssen bei einem derartigen Vergleich die Teilchengrößen der Perlglanzpigmente und der Trägermaterial identisch oder zumindest vergleichbar sein.

Die Partikelgröße der Trägermaterialien, vorzugsweise plättchenförmigen Trägerpartikel, liegt bevorzugt in einem Bereich von 50 bis 2500 $\mu$m, weiter bevorzugt in einem Bereich von 70 bis 1600 $\mu$m, noch weiter bevorzugt in einem Bereich von 80 bis 1000 $\mu$m, besonders bevorzugt in einem Bereich von 100 bis 800 $\mu$m und ganz besonders bevorzugt in einem Bereich von 180 bis 450 $\mu$m.

Annähernd sphärische, linsenförmige oder ungleichmäßig geformte Trägermaterialien weisen vorzugsweise eine Partikelgrößenverteilung auf. Bevorzugt liegen die $D_{50}$-Werte dieser Trägermaterialien in einem Bereich von bis 100 bis 3000 $\mu$m, weiter bevorzugt in einem Bereich von 120 bis 2500 $\mu$m, besonders bevorzugt in einem Bereich von 150 bis 2000 $\mu$m und ganz besonders bevorzugt in einem Bereich von 200 bis 1300 $\mu$m.

Werden plättchenförmige Trägermaterialien in polygonaler Form, vorzugsweise tetragonaler oder hexagonaler Form, eingesetzt, so liegt deren mittlere Dicke vorzugsweise in einem Bereich von 5 bis 150 $\mu$m, weiter bevorzugt in einem Bereich von 6 bis 140 $\mu$m, besonders bevorzugt in einem Bereich von 7 bis 130 $\mu$m und ganz besonders bevorzugt in einem Bereich von 11 bis 120 $\mu$m.

Weist das plättchenförmige Trägermaterial in polygonaler Form eine mittlere Dicke von mindestens 20 $\mu$m auf, so können neben der Oberfläche auch die Schnittkanten des Trägermaterials mit Perlglanzpigmenten belegt sein.

Werden linsenförmige Trägermaterialien eingesetzt, so liegt deren mittlere Dicke vorzugsweise in einem Bereich von 5 bis 500 $\mu$m, weiter bevorzugt in einem Bereich von 6 bis 450 $\mu$m, besonders bevorzugt in einem Bereich von 7 bis 370 $\mu$m und ganz besonders bevorzugt in einem Bereich von 11 bis 350 $\mu$m. Zur Bestimmung der mittleren Dicke der linsenförmigen Trägermaterialien wird der jeweils größte Wert für die Dicke von jedem einzelnen Partikel ermittelt und, wie nachstehend ausgeführt, über wenigstens 100 Partikel gemittelt.

Die mittlere Dicke der Trägermaterialien kann anhand von rasterelektronenmikroskopischen Aufnahmen von Querschliffen bestimmt werden. Hierbei werden bevorzugt wenigstens 100 Einzelpartikel vermessen, um eine aussagekräftige Statistik zu erhalten.

Zur Herstellung der erfindungsgemäßen Kompositpartikel werden Perlglanzpigmente basierend auf nichtmetallischen plättchenförmigen Substraten eingesetzt.

Die nichtmetallischen plättchenförmigen Substrate sind vorzugsweise im Wesentlichen transparent, bevorzugt transparent, d.h. für sichtbares Licht sind sie zumindest teilweise, vorzugsweise im Wesentlichen vollständig, durchlässig.

Die nichtmetallischen plättchenförmigen Substrate können aus der Gruppe, bestehend aus natürlichen Glimmerplättchen, synthetischen Glimmerplättchen, Glasplättchen, $SiO_2$-Plättchen, $Al_2O_3$-Plättchen, BiOCl-Plättchen, $TiO_2$-Plättchen, $Fe_2O_3$-Plättchen, Sericitplättchen, Kaolinplättchen, Graphitplättchen, Talkumplättchen, Polymerplättchen, plättchenförmigen Substraten, die eine anorganisch-organische Mischschicht umfassen, ausgewählt werden. Erfindungsgemäß können als Perlglanzpigmente auch solche verwendet werden, deren Substrate Gemische der vorstehend angegebenen plättchenförmigen Substrate sind.

Bevorzugt werden die nichtmetallischen plättchenförmigen Substrate aus der Gruppe, bestehend aus natürlichen Glimmerplättchen, synthetischen Glimmerplättchen, Glasplättchen, $SiO_2$-Plättchen, $Al_2O_3$-Plättchen und deren Gemische, ausgewählt. Besonders bevorzugt werden die nichtmetallischen plättchenförmigen Substrate aus der Gruppe, bestehend aus natürlichen Glimmerplättchen, synthetischen Glimmerplättchen, Glasplättchen und deren Gemische, ausgewählt. Ganz besonders bevorzugt sind als Substrate Glasplättchen und synthetische Glimmerplättchen und deren Gemische. Insbesondere sind als Substrat Glasplättchen bevorzugt.

Natürliche Glimmerplättchen besitzen im Unterschied zu synthetischen plättchenförmigen transparenten Substraten den Nachteil, dass Verunreinigungen durch eingelagerte Fremdionen den Farbton verändern können, und dass die Oberfläche herstellungsbedingt nicht ideal glatt ist, sondern Unregelmäßigkeiten, wie z.B. Stufen aufweisen kann.

Synthetische Substrate wie beispielsweise Glasplättchen oder synthetische Glimmerplättchen können dagegen glatte Oberflächen sowie eine einheitliche Dicke innerhalb eines einzelnen Substratteilchens sowie vorzugsweise über die Gesamtheit aller Substratteilchen aufweisen. Somit bietet die Oberfläche nur wenig Streuzentren für einfallendes bzw. reflektiertes Licht und ermöglicht so nach Beschichtung dieser plättchenförmigen Substrate höher glänzende und farbstärkere Perlglanzpigmente als mit plättchenförmigem natürlichem Glimmer als Substrat. Als Glasplättchen werden bevorzugt jene verwendet, die nach den in EP 0 289 240 A1, WO 2004/056716 A1 und der WO 2005/063637 A1 beschriebenen Verfahren hergestellt werden. Die als Substrat verwendbaren Glasplättchen können beispielsweise eine Zusammensetzung entsprechend der Lehre der EP 1 980 594 B1 aufweisen.

Das nichtmetallische plättchenförmige Substrat, wie vorstehend beschrieben, kann mit wenigstens einer Schicht oder Beschichtung versehen werden, wobei die wenigstens eine Schicht vorzugsweise Metalloxide, Metalloxidhydrate, Metallhydroxide, Metallsuboxide, Metalle, Metallfluoride, Metalloxyhalogenide, Metallchalcogenide, Metallnitride, Metalloxynitride, Metallsulfide, Metallcarbide oder Mischungen davon umfasst. Gemäß einer bevorzugten Variante besteht die wenigstens eine Schicht oder Beschichtung aus den vorstehend genannten Materialien.

Bevorzugt werden als Schicht oder Beschichtung Metalloxide, Metalloxidhydrate, Metallhydroxide und/ oder deren Mischungen eingesetzt. Besonders bevorzugt werden Metalloxide und Metalloxidhydrate und/oder deren Mischungen eingesetzt. Vorstehend genannte Materialien können entweder als separat voneinander getrennte Schichten oder auch nebeneinander in derselben Schicht vorliegen.

Die Begriffe Schicht oder Beschichtung werden austauschbar verwendet, sofern nicht anders angegeben.

Die Beschichtung kann entweder das Substrat vollständig umhüllen, nur partiell auf dem Substrat vorliegen oder nur dessen obere und/ oder untere Fläche bedecken.

Die wenigstens eine Schicht kann eine optisch wirksame Schicht sein und/ oder als Schutzschicht dienen.

Wird eine hochbrechende Schicht auf ein nichtmetallisches plättchenförmiges Substrat aufgebracht, so liegt der Brechungsindex bei $n \geq 1,8$, bevorzugt bei $n \geq 1,9$ und besonders bevorzugt bei $n \geq 2,0$. Im Falle einer niedrigbrechenden Schicht oder Beschichtung liegt der Brechungsindex bei $n < 1,8$, bevorzugt bei $n < 1,7$ und besonders bevorzugt bei $n < 1,6$.

Als hochbrechende Schicht eignen sich beispielsweise Metalloxide wie Titanoxid, vorzugsweise Titandioxid ($TiO_2$), Eisenoxid, vorzugsweise Eisen(III)oxid ($Fe_2O_3$) und/ oder Eisen(II/III)oxid ($Fe_3O_4$), Zinkoxid, vorzugsweise ZnO, Zinnoxid, vorzugsweise Zinndioxid ($SnO_2$), Zirkoniumoxid, vorzugsweise Zirkoniumdioxid ($ZrO_2$), Antimonoxid vorzugsweise Antimon(III)oxid ($Sb_2O_3$), Magnesiumoxid, vorzugsweise MgO, Ceroxid, vorzugsweise Cer(IV)oxid ($CeO_2$), Cobaltoxid, vorzugsweise Cobalt(II)oxid (CoO) und/oder Cobalt(II/III)oxid ($Co_3O_4$), Chromoxid, vorzugsweise Chrom(III)oxid ($Cr_2O_3$), Kupferoxid, vorzugsweise Kupfer(I)oxid ($Cu_2O$) und/ oder Kupfer(II)oxid (CuO), Vanadiumoxid, vorzugsweise Vanadium(IV)oxid ($VO_2$) und/ oder Vanadium(III)oxid ($V_2O_3$), Metallsulfide wie Zinksulfid, vorzugsweise Zink(II)sulfid (ZnS), Metalloxidhydrate wie Goethit (FeOOH), Titanate wie Calciumtitanat ($CaTiO_3$) oder Eisentitanate, wie z. B. Ilmenit ($FeTiO_3$), Pseudobrookit ($Fe_2TiO_5$) und/ oder Pseudorutil ($Fe_2Ti_3O_9$), dotierte Metalloxide, wie beispielsweise Titandioxid und Zirkoniumdioxid, die mit selektiv absorbierenden Farbmitteln eingefärbt sind, und/ oder deren Gemische. Die zuletzt genannte Einfärbung nicht absorbierender hochbrechender Metalloxide kann z. B. durch Einbau von Farbmitteln in die Metalloxidschicht, durch deren Dotierung mit selektiv absorbierenden Metallkationen oder farbigen Metalloxiden wie Eisen(III)oxid oder durch Überziehen der Metalloxidschicht mit einem ein Farbmittel enthaltenden Film erfolgen.

Bevorzugt umfasst die hochbrechende Schicht Metalloxide, Metallhydroxide und/ oder Metalloxidhydrate. Besonders bevorzugt werden Metalloxide eingesetzt. Ganz besonders bevorzugt werden Titandioxid und/ oder Eisenoxid sowie Mischungen davon verwendet.

Wird mit Titandioxid beschichtet, kann das Titandioxid in der Rutil- oder Anataskristallmodifikation vorliegen. Die Rutilform kann beispielsweise erhalten werden, indem beispielsweise vor Aufbringung der Titandioxidschicht eine Schicht aus Zinndioxid auf das zu beschichtende plättchenförmige Substrat aufgebracht wird. Auf dieser Schicht aus Zinndioxid kristallisiert Titandioxid in der Rutilmodifikation. Das Zinndioxid kann dabei als separate Schicht vorliegen, wobei die Schichtdicke wenige Nanometer, beispielsweise weniger als 10 nm, weiter bevorzugt weniger als 5 nm, noch weiter bevorzugt weniger als 3 nm, betragen kann. Das Zinndioxid kann aber auch mit dem Titandioxid zumindest teilweise in Mischung vorliegen.

Beispiele für niedrigbrechende Schichten sind unter anderem Metalloxide wie Siliziumoxid, vorzugsweise Siliziumdioxid ($SiO_2$), Aluminiumoxid, vorzugsweise $Al_2O_3$, Boroxid, vorzugsweise Bor(III)oxid ($B_2O_3$), Metallfluoride wie Magnesiumfluorid, vorzugsweise $MgF_2$, Aluminiumfluorid, vorzugsweise $AlF_3$, Cerfluorid, vorzugsweise Cer(III)fluorid ($CeF_3$), Calciumfluorid, vorzugsweise $CaF_2$, Metalloxidhydrate wie Aluminiumoxidhydrat AlOOH, Siliziumoxidhydrat, vorzugsweise $SiO_2 \cdot H_2O$ und/ oder deren Gemische.

Vorzugsweise umfasst die niedrigbrechende Schicht Siliziumdioxid.

Ist das nichtmetallische plättchenförmige Substrat mit nur einer einzigen Metalloxidschicht beschichtet, so weist diese vorzugsweise einen hohen Brechungsindex auf. In Abhängigkeit von der geometrischen Metalloxidschichtdicke können derartige Perlglanzpigmente unterschiedliche Farbeffekte bewirken, wie in Tabelle 1 veranschaulicht.

Tabelle 1: Typische Farben und geometrische Schichtdicken von Perlglanzpigmenten

| | Belegung/ geometrische Schichtdicke | Farbe |
|---|---|---|
| Silberweiße Perlglanzpigmente | $TiO_2$: 40 - 60 nm | silber |
| Interferenzpigmente | $TiO_2$: 20 - 40 nm | fahlblau |
| | $TiO_2$: 60 - 80 nm | gelb |
| | $TiO_2$: 80 - 100nm | rot |
| | $TiO_2$: 100 - 140 nm | blau |
| | $TiO_2$: 120 - 160 nm | grün |
| | $TiO_2$: 280 - 320 nm | grün (III. Ordnung) |
| Farbglanzpigmente | $Fe_2O_3$: 35 - 45 nm | bronze |
| | $Fe_2O_3$: 45 - 55 nm | kupfer |
| | $Fe_2O_3$: 55 - 65 nm | rot |
| | $Fe_2O_3$: 65 - 75 nm | rotviolett |
| | $Fe_2O_3$: 75 - 85 nm | rotgrün |
| | $Fe_3O_4$ | schwarz |
| Kombinationspigmente | $TiO_2$/ $Fe_2O_3$ | Goldtöne |
| | $TiO_2$/ $Cr_2O_3$ | grün |
| | $TiO_2$/ Berliner Blau | tiefblau |

Besteht das plättchenförmige transparente Substrat aus synthetischem Glimmer sind derartige Perlglanzpigmente z. B. unter dem Handelsnamen Symic kommerziell erhältlich (Fa. Eckart, 91235 Hartenstein, Deutschland). Mit $TiO_2$ und/ oder Eisenoxid beschichtete $Al_2O_3$-Flakes und entsprechend beschichtete $SiO_2$-Flakes werden beispielsweise unter den Handelsnamen Xirallic bzw. Colorstream angeboten (Fa. Merck KGaA, Darmstadt, Deutschland). Mit $TiO_2$ und/ oder Eisenoxid beschichtete Glasplättchen werden z. B. unter dem Namen Luxan (Fa. Eckart), unter der Bezeichnung Firemist (Fa. BASF SE, Ludwigshafen, Deutschland) oder unter der Bezeichnung Miraval (Fa. Merck) angeboten.

Perlglanzpigmente, basierend auf künstlichen Substraten und charakterisiert durch die Kennzahlen $D_{10}$, $D_{50}$ und $D_{90}$ aus der Summenhäufigkeitsverteilung der volumengemittelten Größenverteilungsfunktion mit einem Span $\Delta D$ = $(D_{90} - D_{10})/D_{10}$ von 0,7 bis 1,4 zeichnen sich gemäß WO 2009/103322 A1 durch ihre hohe Farbreinheit aus. Diese Perlglanzpigmente werden gemäß einer bevorzugten Variante der Erfindung bevorzugt verwendet.

Die nichtmetallischen plättchenförmigen Substrate können auch mit einem mehrlagigen Schichtaufbau mit oder bestehend aus Metalloxid, Metallhydroxid, Metallsuboxid und/ oder Metalloxidhydrat beschichtet sein, wobei die Reihenfolge der Schichten variabel sein kann. Bevorzugt ist hierbei eine Schichtenfolge, bei dem wenigstens eine hochbrechende Schicht und wenigstens eine niedrigbrechende Schicht in alternierender Weise auf einem Substrat angeordnet sind. Bei der alternierenden Anordnung ist es auch möglich, ein oder mehrere hochbrechende Schichten unmittelbar übereinander und nachfolgend ein oder mehrere niedrigbrechende Schichten unmittelbar übereinander anzuordnen. Wesentlich ist jedoch, dass in dem Schichtaufbau hoch- und niedrigbrechende Schichten vorkommen. Vorzugsweise sind aufgehend vom plättchenförmigen Substrat wenigstens eine hoch-, niedrig- und wiederum hochbrechende Schicht angeordnet, was in Perlglanzpigmenten mit besonders intensiven Interferenzfarben resultiert. Die Interferenzfarbe kann hierbei in Abhängigkeit von Schichtaufbau und -dicken silbern oder nicht silbern sein.

Bei einer Ausführungsform können die erfindungsgemäßen Kompositpartikel Mischungen verschiedener Perlglanzpigmente umfassen. Beispielsweise können auf dem Trägermaterial Perlglanzpigmente basierend auf z. B. plättchenförmigem synthetischen Glimmer unterschiedlicher Partikelgröße oder mit verschiedener Beschichtung vorliegen. Daneben können die Trägermaterialien auch mit Perlglanzpigmenten basierend auf unterschiedlichen Substraten, wie z. B. Glasplättchen und plättchenförmigem synthetischem Glimmer, mit gegebenenfalls verschiedenen Beschichtungen, belegt sein. Ein Trägermaterial kann somit beispielsweise sowohl silberne als auch blaue Perlglanzpigmente gleicher oder verschiedener Partikelgröße sowie gegebenenfalls Perlglanzpigmente basierend auf unterschiedlichen plättchenförmigen Substraten umfassen.

Die $D_{50}$-Werte der erfindungsgemäß einzusetzenden Perlglanzpigmente liegen bevorzugt in einem Bereich von 3 bis 350 µm, besonders bevorzugt in einem Bereich von 5 bis 300 µm und ganz besonders bevorzugt in einem Bereich von 6 bis 250 µm.

Die Größenverteilungskurven der Trägermaterialien und der Perlglanzpigmente werden mit einem Gerät der Fa. Malvern Instruments GmbH, 71083 Herrenberg, Deutschland, (Mastersizer 2000) gemäß Herstellerangaben bestimmt. Hierzu werden die Trägermaterialien bzw. die Perlglanzpigmente als wässrige Suspension mehrfach, vorzugsweise dreifach, vermessen. Aus den einzelnen Messergebnissen werden sodann Mittelwerte gebildet. Die Auswertung der Streulichtsignale erfolgt dabei nach der Mie-Theorie. Die $D_{10}$-, $D_{50}$- und $D_{90}$-Werte werden mittels Laserbeugung bestimmt. Dies bedeutet, dass 10 %, 50 % bzw. 90 % der Trägermaterialien, vorzugsweise Trägerpartikel, bzw. Effektpigmente einen volumenbezogenen Durchmesser aufweisen, der gleich oder kleiner als der jeweils angegebene Wert ist.

Bei einer weiteren Ausführungsform können anstelle von und/oder neben Perlglanzpigmenten auch erfindungsgemäße Kompositpartikel auf ein Trägermaterial, vorzugsweise Trägerpartikel, aufgebracht werden. Gemäß einer weiteren Ausführungsform können Kompositpartikel, basierend auf einem plättchenförmigen hexagonalen Trägermaterial, und ein annähernd sphärisches Trägermaterial miteinander verbunden werden. Dies geschieht z. B. durch Erwärmen des annähernd sphärischen Trägermaterials unter nachfolgender Zumischung des Kompositpartikels.

Werden Schwarz-Weiß-Deckungskarten mit den erfindungsgemäßen Kompositpartikeln beispielsweise im Siebdruckverfahren bedruckt, so nimmt ein Betrachter zunächst einen hohen Glanz- und Glitzereffekt wahr. Außerhalb des Glanzwinkels erscheinen diese Schwarz-Weiß-Deckungskarten aufgrund der Transparenz der erfindungsgemäßen Kompositpartikel nicht dunkel oder schwarz, wie dies beispielsweise bei mit deckenden Glittern bedruckten Schwarz-Weiß-Deckungskarten der Fall wäre. Objektiv kann dieser winkelabhängige Glanzeffekt bzw. sogenannte Hell-/ Dunkelflop durch den Flopindex beschrieben werden. Der Flopindex wird nach Alman folgendermaßen definiert (S. Schellenberger, M. Entenmann, A. Hennemann, P. Thometzek, Farbe und Lack, 04/2007, S. 130):

$$\text{Flopindex} = 2{,}69 \cdot (L_{E1} - L_{E3})^{1,11}/L_{E2}^{0,86}$$

mit $L_{E1}$ Helligkeit des glanznahen Messwinkels (E1 = 15° zum Glanzwinkel), $L_{E2}$ Helligkeit des Messwinkels zwischen glanznahem und glanzfernen Winkel (E2 = 45° zum Glanzwinkel) und $L_{E3}$ Helligkeit des glanzfernen Messwinkels (E3 = 110° zum Glanzwinkel). Je größer der Zahlenwert des Flopindexes ist, desto stärker kommt die winkelabhängige Helligkeitsänderung, also der Hell-/Dunkelflop zum Ausdruck.

Unter direkter Beleuchtung erscheinen Schwarz-Weiß-Deckungskarten, welche mit den erfindungsgemäßen Kompositpartikeln beispielsweise im Siebdruckverfahren bedruckt oder, wie in Bronzierungsverfahren, bestreut wurden, stark glitzernd. Unter diffuser Beleuchtung werden diese Schwarz-Weiß-Deckungskarten als körnig empfunden. Die Körnigkeit wird mit dem Gerät Byk mac, Fa. Byk-Gardner, bestimmt. Zur Bewertung der Körnigkeit nimmt die hochauflösende CCD-Kamera ein Bild bei diffuser Beleuchtung auf, die durch zwei weiß beschichtete Halbkugeln erzeugt wird. Das Bild wird mit Hilfe des Histogrammes der Helligkeitsstufen analysiert, wobei die Homogenität der hellen und dunklen Flächen in einem Körnigkeitswert zusammengefasst wird. (Byk-Gardner, Qualitätskontrolle für Lacke und Kunststoffe, 2011/2012, S. 98).

Die erfindungsgemäßen Kompositpartikel sind erhältlich durch ein Verfahren, welches folgenden Schritt umfasst:

Mischen von Trägermaterial, vorzugsweise Trägerpartikeln, und wenigstens einem Perlglanzpigment, wobei das Trägermaterial eine wenigstens teilweise erweichte Oberfläche aufweist.

Gemäß einer bevorzugten Variante der Erfindung erfolgt das Mischen von Trägermaterial, vorzugsweise Trägerpartikeln, und wenigstens einem Perlglanzpigment unter Erwärmen, wobei die Oberfläche des Trägermaterials, vorzugsweise der Trägerpartikel, wenigstens teilweise erweicht.

Alternativ kann das Trägermaterial, vorzugsweise die Trägerpartikel, auch in einem ersten Schritt erwärmt und sodann Perlglanzpigment zugemischt werden.

Vorzugsweise erfolgt das Erwärmen des Trägermaterials, bevorzugt der Trägerpartikel, unter Bewegung des Trägermaterials.

Die Bewegung des Trägermaterials kann in einem Mischer oder in einer Wirbelschicht erfolgen.

Gemäß einer bevorzugten Variante der Erfindung umfasst das erfindungsgemäße Verfahren folgende Schritte:

(a) Mischen von Trägermaterial und wenigstens einem Perlglanzpigment unter Bereitstellen einer Mischung,
(b) Erwärmen der in Schritt (a) erhaltenen Mischung unter Erhalt der Kompositpartikel,
(c) optionales Klassieren der in Schritt (b) erhaltenen Kompositpartikel.

Gemäß einer bevorzugten Variante des erfindungsgemäßen Verfahrens erfolgt das Mischen in Schritt (a) und vorzugsweise auch das Erwärmen in Schritt (b) in einem Mischer, in dem das Trägermaterial, bevorzugt die Trägerpartikel, und das wenigstens eine Perlglanzpigment mit einer Umfangsgeschwindigkeit von wenigstens 3 m/s, weiter bevorzugt wenigstens 4 m/s, noch weiter bevorzugt von wenigstens 6 m/s, bewegt werden.

Das Erwärmen in Schritt (b) erfolgt in Abhängigkeit von den thermischen Erweichungseigenschaften des Trägermaterials. Vorzugsweise erfolgt bei Trägermaterialien aus oder mit Kunststoffen das Erweichen in einem Temperaturbereich von 40 bis 230° C, weiter bevorzugt von 45° C bis 190° C, noch weiter bevorzugt von 50 bis 170° C.

Ein wesentlicher Vorteil dieses Herstellungsverfahrens liegt in seiner einfachen Ausführbarkeit. Zur Haftung der Perlglanzpigmente auf dem Trägermaterial muss letzteres weder in aufwändiger Weise vorbehandelt werden, noch muss ein zusätzlicher Haftvermittler zugegeben werden. Ein zusätzlicher Haftvermittler ist erfindungsgemäß nicht gewünscht, da die optischen Eigenschaften des Kompositpartikels in unerwünschter Weise verändert werden können. Des Weiteren besteht die Gefahr von Inkompatibilitäten des Haftvermittlers mit Anwendungsmedien. Schließlich ist ein separater Beschichtungsschritt mit einem Haftvermittler aufwändig und kostenintensiv, insbesondere bei einem Massenprodukt.

Die Bindung zwischen Trägermaterial, vorzugsweise Trägerpartikel, und dem wenigstens einen Perlglanzpigment wird ausschließlich durch spezifische Adhäsion erreicht, wobei die Bindung an die wenigstens teilweise erweichte Trägermaterialoberfläche erfolgt.

Bei einer weiteren Ausführungsform der Erfindung umfasst das Verfahren folgende Schritte:

(a) Mischen von Trägermaterial und wenigstens einem Perlglanzpigment unter Bereitstellen einer Mischung,
(b) Einbringen von Lösemittel in die in Schritt (a) erhaltene Mischung,
(c) Verflüchtigen des Lösemittels aus der in Schritt (b) mit Lösemittel behandelten Mischung unter Erhalt der Kompositpartikel,
(d) optionales Klassieren, vorzugsweise Schutzklassieren, der in Schritt (b) oder (c) erhaltenen Kompositpartikel.

Gemäß einer Weiterbildung der Erfindung kann Schritt (b) das Lösemittel als Lösemitteldampf zugeführt werden.

Gemäß einer Weiterbildung der Erfindung kann Schritt (c) unter Erwärmen erfolgen, wodurch die Verflüchtigung des Lösemittels begünstigt wird.

Bei dieser Variante des erfindungsgemäßen Verfahrens erfolgt das wenigstens teilweise Erweichen der Oberfläche des Trägermaterials, vorzugsweise der Trägermaterialpartikel, durch den Einfluss des Lösemittels, wodurch die Perlglanzpigmente an die Oberfläche binden.

Die erfindungsgemäßen Kompositpartikel liegen in nichtstaubender Form vor und können zur Kunststoffeinfärbung, in Nagellacken oder in der graphischen Industrie zum Einsatz kommen. Insbesondere können die erfindungsgemäßen Kompositpartikel zur Veredelung von Tapeten oder Druckerzeugnissen, wie z.B. Grußkarten oder Faltschachteln, eingesetzt werden. Diese Veredelung kann zum einen durch Bronzierung, zum anderen in Sieb- oder Tiefdruckverfahren erfolgen. Einer bereits bedruckten Tapete oder einem bereits bedruckten Druckerzeugnis kann nach Bronzierung mit den erfindungsgemäßen Kompositpartikeln ein glitzerndes Aussehen verliehen werden. Auch können die erfindungsgemäßen Kompositpartikel in Sieb- oder Tiefdruckverfahren auf bereits bedruckte Tapeten oder Druckerzeugnisse verdruckt werden. Der Druckvorgang mit den erfindungsgemäßen Kompositpartikeln kann hierbei auch direkt beim Verdrucken eines gewünschten Motivs erfolgen und muss nicht zwangsläufig auf einer bereits bedruckten Tapete oder Druckerzeugnis erfolgen.

Vorzugsweise werden Kompositpartikel, basierend auf plättchenförmigen Trägermaterialien, mittels Bronzierung, Tief- oder Siebdruck appliziert. Kompositpartikel, basierend auf annähernd sphärischen, linsenförmigen oder irregulär geformten Trägermaterialien, werden bevorzugt mittels Bronzierung oder Aufstreueinrichtungen mit gravierten Schöpfwalzen appliziert. Die Schöpfwalzen dienen insbesondere dazu, Kompositpartikel mit einer mittleren Partikelgröße $D_{50}$ von 100 bis 2000 $\mu$m kontrolliert auf das jeweilige Substrat zu applizieren.

Die Erfindung betrifft ferner folgende bevorzugte Ausführungsformen:

Kompositpartikel, umfassend ein Trägermaterial, vorzugsweise ein plättchenförmiges hexagonales Trägerpartikel, sowie wenigstens ein Perlglanzpigment, basierend auf Glasplättchen, wobei das Trägermaterial und das wenigstens eine Perlglanzpigment ohne zusätzliches Haftmittel aneinander gebunden vorliegen.

Kompositpartikel, umfassend ein Trägermaterial, vorzugsweise ein plättchenförmiges polygonales, vorzugsweise hexagonales Trägerpartikel, sowie wenigstens ein Perlglanzpigment, basierend auf Glasplättchen oder synthetischen Glimmerplättchen, wobei das Trägermaterial und das wenigstens eine Perlglanzpigment ohne zusätzliches Haftmittel aneinander gebunden vorliegen und der Anteil an Perlglanzpigment in einem Bereich von 0,1 bis 20 Gew.-%, bevorzugt in einem Bereich von 0,2 bis 15 Gew.-%, weiter bevorzugt in einem Bereich von 0,3 bis 12 Gew.-%, bezogen auf das Gesamtgewicht des Kompositpartikels, liegt.

Kompositpartikel, umfassend ein Trägermaterial, vorzugsweise ein plättchenförmiges polygonales, vorzugsweise hexagonales Trägerpartikel aus Polyethylenterephthalat (PET), sowie wenigstens ein Perlglanzpigment, basierend auf Glasplättchen, erhalten durch Mischen von Trägermaterial und Perlglanzpigment unter Erwärmung.

Kompositpartikel, umfassend ein Trägermaterial, vorzugsweise ein plättchenförmiges polygonales, vorzugsweise hexagonales Trägerpartikel aus mindestens zwei Folienschichten sowie wenigstens ein Perlglanzpigment, basierend auf Glasplättchen, wobei das Trägermaterial und das wenigstens eine Perlglanzpigment ohne zusätzliches Haftmittel aneinander gebunden vorliegen.

Kompositpartikel, umfassend ein Trägermaterial, vorzugsweise ein plättchenförmiges polygonales, vorzugsweise hexagonales Trägerpartikel aus mindestens drei flächig übereinander angeordneten Folienschichten, wobei auf den beiden außenliegenden Folienschichten des Trägermaterials jeweils wenigstens ein Perlglanzpigment ohne zusätzliches Haftmittel an das Trägermaterial gebunden vorliegt.

Kompositpartikel, umfassend ein Trägermaterial aus Polyvinylacetat, vorzugsweise ein annähernd sphärisches Trägerpartikel, sowie wenigstens ein Perlglanzpigment, basierend auf Glasplättchen oder synthetischen Glimmerplättchen, wobei das Trägermaterial und das wenigstens eine Perlglanzpigment ohne zusätzliches Haftmittel aneinander gebunden vorliegen.

Kompositpartikel, umfassend ein Trägermaterial aus vorzugsweise Polyvinylbutyral, vorzugsweise ein linsenförmiges Trägerpartikel, vorzugsweise mit einer Teilchengröße von $\leq 1,5$ mm, sowie wenigstens ein Perlglanzpigment, basierend auf Glasplättchen oder synthetischen Glimmerplättchen, wobei das Trägermaterial und das wenigstens eine Perlglanzpigment ohne zusätzliches Haftmittel aneinander gebunden vorliegen.

Kompositpartikel, umfassend ein erstes Trägermaterial, ohne Haftmittel gebunden an ein zweites, vorzugsweise sphärisches, Trägermaterial, wobei das erste Trägermaterial des Kompositpartikels und das zweite Trägermaterial voneinander verschieden sind. Das erste und das zweite Trägermaterial sind vorzugsweise in der geometrischen Form voneinander verschieden. Alternativ oder kumulativ können sich das erste Trägermaterial und das zweite Trägermaterial auch in der stofflichen Beschaffenheit, vorzugsweise Zusammensetzung, unterscheiden.

Kompositpartikel, basierend auf einem ersten, plättchenförmigen polygonalen, vorzugsweise hexagonalen, Trägerpartikel, welches mit Perlglanzpigmenten, basierend auf Glasplättchen oder synthetischen Glimmerplättchen, belegt ist und wobei das Kompositpartikel ohne Haftmittel an ein zweites Trägermaterial, vorzugsweise ein annähernd sphärisches Trägerpartikel, vorzugsweise aus Polyvinylacetat, mit einer bevorzugten Teilchengröße von $\leq 2$ mm, vorzugsweise mit einer Teilchengröße von 0,5 bis $\leq 1,5$ mm, gebunden ist. Gemäß einer weiteren Ausführungsform der Erfindung können optional auch auf dem zweiten, vorzugsweise annähernd sphärischen, Trägerpartikel, auch Perlglanzpigmente vorhanden sein.

Beschichtungsmaterial wie ein Kosmetikum, vorzugsweise ein Nagellack, welches Kompositpartikel umfasst.

Die nachfolgenden Beispiele und Figuren dienen der näheren Beschreibung der Erfindung und sollen in keinerlei Hinsicht einschränkend sein. Alle Angaben sind als Gew.-% zu verstehen.

**Figuren**

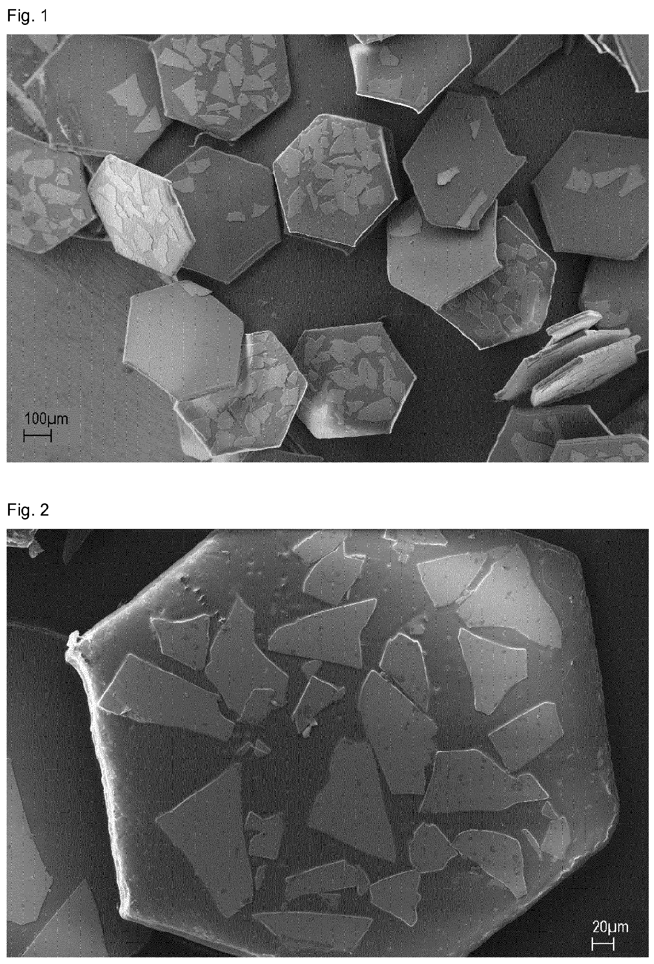

Figur 1 zeigt eine rasterelektronenmikroskopische (REM)-Aufnahme des Kompositpartikels aus Beispiel 2 in 50-facher Vergrößerung.

Figur 2 zeigt eine rasterelektronenmikroskopische (REM)-Aufnahme des Kompositpartikels aus Beispiel 2 in 200-facher Vergrößerung.

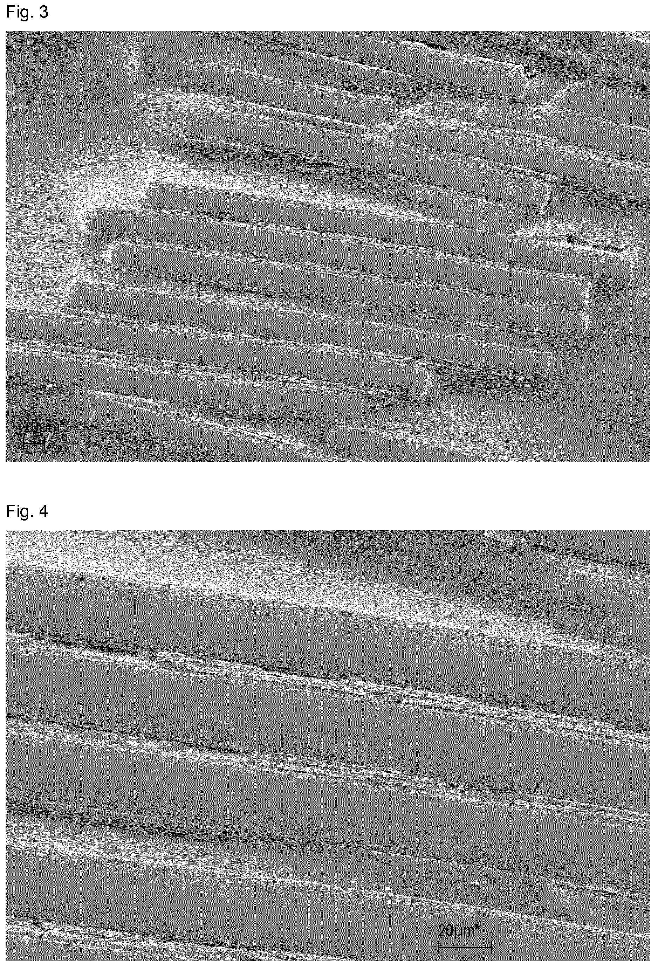

Figur 3 zeigt eine rasterelektronenmikroskopische (REM)-Aufnahme des Kompositpartikels aus Beispiel 2 in 200-facher Vergrößerung.

Figur 4 zeigt eine rasterelektronenmikroskopische (REM)-Aufnahme des Kompositpartikels aus Beispiel 2 in 500-facher Vergrößerung.

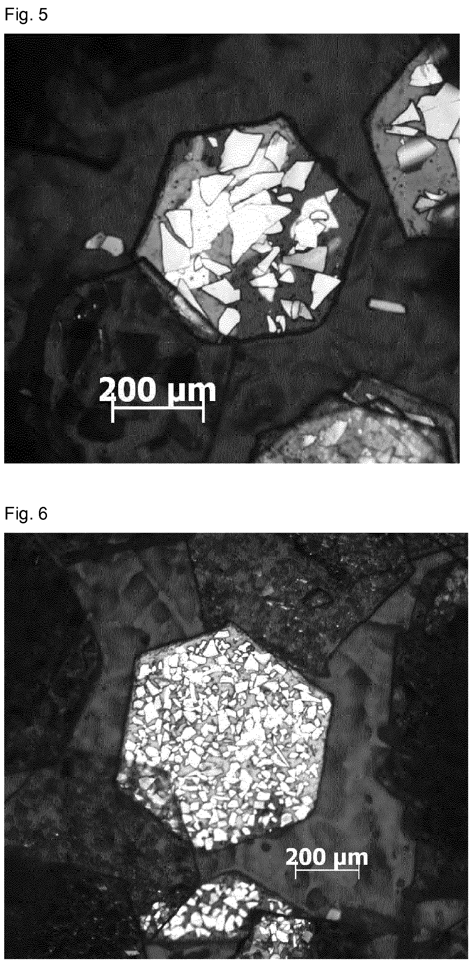

Figur 5 zeigt eine lichtmikroskopische Aufnahme des Kompositpartikels aus Beispiel 1 (Aufstreuung auf Schwarz-Weiß-Deckungskarte) in 50-facher Vergrößerung.

Figur 6 zeigt eine lichtmikroskopische Aufnahme des Kompositpartikels aus Beispiel 3 (Aufstreuung auf Schwarz-Weiß-Deckungskarte) in 50-facher Vergrößerung.

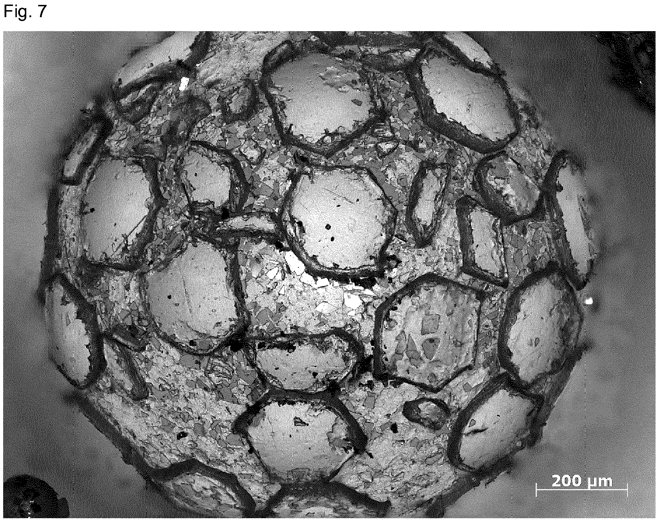

Figur 7 zeigt eine rasterelektronenmikroskopische (REM)-Aufnahme des Kompositpartikels aus Beispiel 12 in 100-facher Vergrößerung.

**Beispiele**

**I Herstellung der erfindungsgemäßen Kompositpartikel**

Beispiel 1

94 Gew.-% PET Glitter (Crystal Clear 0.008" 45.008E, einseitig beschichtet zur Vermeidung der Bindung von Perlglanzpigmenten an die beschichtete Seite, Fa. RJA plastics GmbH, 07989 Teichwolframsdorf, Deutschland, Teilchengröße gemäß Herstellerangaben: 200 $\mu$m) wurden zusammen mit 5 Gew.-% Luxan C001 ($D_{50}$ = 30 - 35 $\mu$m) und 1 Gew.-% Luxan C261 ($D_{50}$ = 28 - 33 $\mu$m), beide Fa. Eckart, in einen Labor-Schnellmischer System Papenmeier, Fa. Gebrüder Lödige Maschinenbau GmbH, 33102 Paderborn, Deutschland, gegeben und 1 Minute bei einer Umfangsgeschwindigkeit von 8 m/s gemischt. Anschließend wurde für 14 Minuten eine Umfangsgeschwindigkeit von 13 m/s und danach für 7 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 2

94 Gew.-% PET Glitter (Crystal Clear 0.016" 45.016E, einseitig beschichtet zur Vermeidung der Bindung von Perlglanzpigmenten an die beschichtete Seite, Fa. RJA plastics GmbH, Teilchengröße gemäß Herstellerangaben: 400 $\mu$m) wurden zusammen mit 5 Gew.-% Luxan E001 ($D_{50}$ = 85 - 90 $\mu$m) und 1 Gew.-% Luxan E261 ($D_{50}$ = 80 - 85 $\mu$m), beide Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 19 Minuten eine Umfangsgeschwindigkeit von 13 m/s und danach für 7 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 3

94 Gew.-% PET Glitter (Crystal Clear 0.025" 45.025E, einseitig beschichtet zur Vermeidung der Bindung von Perlglanzpigmenten an die beschichtete Seite, Fa. RJA plastics GmbH, Teilchengröße gemäß Herstellerangaben: 600 $\mu$m) wurden zusammen mit 5 Gew.-% Luxan C001 ($D_{50}$ = 30 - 35) und 1 Gew.-% Luxan C261 ($D_{50}$ = 28 - 33), beide Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 22 Minuten eine Umfangsgeschwindigkeit von 13 m/s und danach für eine Minute eine Umfangsgeschwindigkeit von 8 m/s beibehalten. Im Anschluss wurde der Mischvorgang für eine Minute unterbrochen bevor für eine Minute eine Umfangsgeschwindigkeit von 8 m/s eingestellt wurde. Diese Unterbrechung für eine Minute und anschließende Einstellung einer Umfangsgeschwindigkeit von 8 m/s für eine Minute wurde insgesamt dreimal wiederholt. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 4

95 Gew.-% PET Glitter (Crystal Clear 0.016" 45.016E, einseitig beschichtet zur Vermeidung der Bindung von Perlglanzpigmenten an die beschichtete Seite, Fa. RJA plastics GmbH) wurden zusammen mit 5 Gew.-% Luxan C261 ($D_{50}$ = 80 - 85 $\mu$m), Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 19 Minuten eine Umfangsgeschwindigkeit von 13 m/s und danach für 5 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 5

95 Gew.-% PET Glitter (Crystal Clear 0.016" 45.016E, einseitig beschichtet zur Vermeidung der Bindung von Perlglanzpigmenten an die beschichtete Seite, Fa. RJA plastics GmbH) wurden zusammen mit 5 Gew.-% Symic C001, Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 25 Minuten eine Umfangsgeschwindigkeit von 13 m/s und danach für 5 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 6

98 Gew.-% PVC Granulat (Optigran TG101, Fa. JSC Veika, Vilnius, Litauen) wurden zusammen mit 2 Gew.-% Luxan E001 ($D_{50}$ = 85 - 90 $\mu$m), Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 10 Minuten eine Umfangsgeschwindigkeit von 13 m/s und danach für 5 Minuten eine Umfangsgeschwindigkeit von 8 m/s beibehalten. Vor und während des gesamten Mischvorgangs wurde der Mischbehälter mit einer Vorlauftemperatur von 105° C beheizt. Nach Entleeren des Mischbehälters wurden die so erhaltenen Kompositpartikel auf Raumtemperatur abgekühlt und über ein Vibrationssieb der Maschenweite 2000 $\mu$m schutzklassiert.

Beispiel 7

97 Gew.-% PVC Granulat (Optideco 7103 EK1, Fa. JSC VEIKA) wurden zusammen mit 3 Gew.-% Luxan F001 ($D_{50}$ = 200 - 210 $\mu$m), Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 5 Minuten eine Umfangsgeschwindigkeit von 13 m/s und danach für 5 Minuten eine Umfangsgeschwindigkeit von 8 m/s beibehalten. Vor und während des gesamten Mischvorgangs wurde der Mischbehälter mit einer Vorlauftemperatur von 120° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 8

95 Gew.-% Polyvinylbutyralgranulat (PVB-Granulat B75H 0,4-0,7 mm, Fa. Polikom, Broshniv-Osada, Ukraine) wurden zusammen mit 4 Gew.-% Luxan E001 ($D_{50}$ = 85 - 90 $\mu$m) und 1 Gew.-% Luxan E261 ($D_{50}$ = 80 - 85 $\mu$m), beide Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 17 Minuten eine Umfangsgeschwindigkeit von 13 m/s und danach für fünf Minuten eine Umfangsgeschwindigkeit von 8 m/s beibehalten. Vor und während des gesamten Mischvorgangs wurde der Mischbehälter mit einer Vorlauftemperatur von 120°C beheizt. Nach Entleeren des Mischbehälters wurden die so erhaltenen Kompositpartikel auf Raumtemperatur abgekühlt und über ein Vibrationssieb der Maschenweite 2000 $\mu$m schutzklassiert.

Beispiel 9

99 Gew.-% Vinylacetat Homopolymer Kugeln (VINNAPERL 20, Fa. Wacker Chemie AG, Burghausen, Deutschland, Teilchengrößenverteilung gemäß Herstellerangaben 0,1 - 0,8 mm) wurden zusammen mit 1 Gew.-% Phoenix PX 4522, Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 8 Minuten eine Umfangsgeschwindigkeit von 13 m/s und danach für drei Minuten eine Umfangsgeschwindigkeit von 8 m/s beibehalten. Vor und während des gesamten Mischvorgangs wurde der Mischbehälter mit einer Vorlauftemperatur von 90°C beheizt. Nach Entleeren des Mischbehälters wurden die so erhaltenen Kompositpartikel auf Raumtemperatur abgekühlt und über ein Vibrationssieb der Maschenweite 2000 $\mu$m schutzklassiert.

Beispiel 10

99 Gew.-% Vinylacetat Homopolymer Kugeln (VINNAPERL 20, Fa. Wacker Chemie AG, Burghausen, Deutschland, Teilchengrößenverteilung gemäß Herstellerangaben 0,1 - 0,8 mm) wurden zusammen mit 1 Gew.-% Luxan C001, Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 8 Minuten eine Umfangsgeschwindigkeit von 13 m/s und danach für drei Minuten eine Umfangsgeschwindigkeit von 8 m/s beibehalten. Vor und während des gesamten Mischvorgangs wurde der Mischbehälter mit einer Vorlauftemperatur von 90° C beheizt. Nach Entleeren des Mischbehälters wurden die so erhaltenen Kompositpartikel auf Raumtemperatur abgekühlt und über ein Vibrationssieb der

Maschenweite 2000 $\mu$m schutzklassiert.

Beispiel 11

94 Gew.-% PET Glitter (Crystal Black 0.016" 104.016E, einseitig beschichtet zur Vermeidung der Bindung von Perlglanzpigmenten an die beschichtete Seite, Fa. RJA plastics GmbH, Teilchengröße gemäß Herstellerangaben: 400 $\mu$m) wurden zusammen mit 6 Gew.-% Luxan E261 ($D_{50}$ = 80 - 85 $\mu$m), Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 19 Minuten eine Umfangsgeschwindigkeit von 13 m/s und danach für 7 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 12

94 Gew.-% PET Glitter (Crystal Yellow 0.016" 50.015E, einseitig beschichtet zur Vermeidung der Bindung von Perlglanzpigmenten an die beschichtete Seite, Fa. RJA plastics GmbH, Teilchengröße gemäß Herstellerangaben: 400 $\mu$m) wurden zusammen mit 6 Gew.-% Luxan E221 ($D_{50}$ = 80 - 85 $\mu$m), Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 19 Minuten eine Umfangsgeschwindigkeit von 13 m/s und danach für 7 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Beispiel 13

89 Gew.-% Vinylacetat Homopolymer Kugeln (VINNAPERL 20, Fa. Wacker Chemie AG, Burghausen, Deutschland, Teilchengrößenverteilung gemäß Herstellerangaben 0,1 -0,8 mm) wurden zusammen mit 10 Gew.-% PET Glitter (Crystal Clear 0.008", Fa. RJA plastics GmbH, vor dem Zuschneiden der Folie beidseitig beschichtet mit Polyvinylbutyral mittels Lösemittel-Tiefdruck mit Zylinderkonfiguration von 70 Linien/cm und einem Volumen von 14 cm$^3$/m$^2$, Poylvinylbutyral 25 Gew.-% in Ethanol) in einen Labor-Schnellmischer System Papenmeier, Fa. Lödige, gegeben und eine Minute bei einer Umfangsgeschwindigkeit von 8 m/s gemischt.
Anschließend wurde für 26 Minuten eine Umfangsgeschwindigkeit von 13 m/s beibehalten. Anschließend wurde der Mischvorgang gestoppt und 1 Gew.-% Luxan C001 ($D_{50}$ = 30 - 35 $\mu$m), Fa. Eckart, zugegeben. Danach wurde 20 Minuten bei einer Umfangsgeschwindigkeit von 13 m/s gemischt. Vor dem Mischvorgang und während des Mischvorgangs, bis zu der Zugabe von Luxan C001, wurde der Mischbehälter mit einer Vorlauftemperatur von 110 bis 125° C beheizt. Anschließend wurde bis zum Ende des Mischvorgangs der Mischbehälter mit einer Vorlauftemperatur von 125 bis 135° C beheizt. Nach Entleeren des Mischbehälters wurden die so erhaltenen Kompositpartikel auf Raumtemperatur abgekühlt und über ein Vibrationssieb der Maschenweite 2000 $\mu$m schutzklassiert.

Beispiel 14

90 Gew.-% PET Glitter (Crystal Clear 0.08", Fa. RJA plastics GmbH, vor dem Zuschneiden der Folie beidseitig beschichtet mit Polyvinylbutyral mittels Lösemittel-Tiefdruck mit Zylinderkonfiguration von 70 Linien/cm und einem Volumen von 14 cm$^3$/m$^2$, Poylvinylbutyral 25 Gew.-% in Ethanol) wurden zusammen mit 10 Gew.-% LUXAN C001, Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 12 Minuten mit einer Umfangsgeschwindigkeit von 13 m/s gemischt. Danach wurde für 6 Minuten eine Umfangsgeschwindigkeit von 8 m/s beibehalten. Vor und während des Mischvorgangs wurde der Mischbehälter mit einer Vorlauftemperatur von 135° C beheizt.
In einem zweiten Verfahrensschritt wurden dann 10 Gew.-% der so erhaltenen Mischung mit 90 Gew.-% Vinylacetat Homopolymer Kugeln (VINNAPERL 20, Fa. Wacker Chemie AG, Burghausen, Deutschland, Teilchengrößenverteilung 0,1 - 0,8 mm) in einen Labor-Schnellmischer System Papenmeier, Fa. Lödige, gegeben und eine Minute bei einer Umfangsgeschwindigkeit von 8 m/s gemischt. Danach wurde für 29 Minuten eine Umfangsgeschwindigkeit von 13 m/s beibehalten. Anschließend wurde fünf Minuten eine Umfangsgeschwindigkeit von 8 m/s beibehalten. Vor und während des Mischvorgangs wurde der Mischbehälter mit einer Vorlauftemperatur von 100° C beheizt.
Nach Entleeren des Mischbehälters wurden die so erhaltenen Kompositpartikel auf Raumtemperatur abgekühlt und über ein Vibrationssieb der Maschenweite 2000 $\mu$m schutzklassiert.

Beispiel 15

88 Gew.-% PET Glitter (Crystal Clear 0.016" 45.016E, Fa. RJA plastics GmbH, Teilchengröße gemäß Herstellerangaben: 400 $\mu$m, vor dem Zuschneiden der Folie beidseitig beschichtet mit Polyvinylbutyral mittels Lösemittel-Tiefdruck mit Zylinderkonfiguration von 70 Linien/cm und einem Volumen von 14 cm$^3$/m$^2$, Poylvinylbutyral 25 Gew.-% in Ethanol) wurden zusammen mit 10 Gew.-% Luxan E001 ($D_{50}$ = 85 - 90 $\mu$m) und 2 Gew.-% Luxan E261 ($D_{50}$ = 80 - 85 $\mu$m), beide Fa. Eckart, in einen Labor-Schnellmischer System Papenmeier, Fa. Lödige, gegeben und eine Minute bei einer Umfangsgeschwindigkeit von 8 m/s gemischt. Anschließend wurde für 19 Minuten eine Umfangsgeschwindigkeit von 13 m/s und danach für 7 Minuten eine Umfangsgeschwindigkeit von 8 m/s beibehalten. Vor und während des gesamten Mischvorgangs wurde der Mischbehälter mit einer Vorlauftemperatur von 150° C beheizt. Nach Entleeren des Mischbehälters wurden die so erhaltenen Kompositpartikel auf Raumtemperatur abgekühlt und über ein Vibrationssieb der Maschenweite 1000 $\mu$m schutzklassiert.

Vergleichsbeispiel 1

Polyester Hexagon Glitter Silber 0.008", Fa. RJA plastics GmbH.

Vergleichsbeispiel 2

Polyester Hexagon Glitter Silber 0.016", Fa. RJA plastics GmbH.

Vergleichsbeispiel 3

Polyester Hexagon Glitter Silber 0.025", Fa. RJA plastics GmbH.

Vergleichsbeispiel 4

Crystal Clear 0.008" 45.008E, einseitig beschichtet, Fa. RJA plastics GmbH.

Vergleichsbeispiel 5

Crystal Clear 0.016" 45.016E, einseitig beschichtet, Fa. RJA plastics GmbH.

Vergleichsbeispiel 6

Crystal Clear 0.025" 45.025E, einseitig beschichtet, Fa. RJA plastics GmbH.

Vergleichsbeispiel 7

90 Gew.-% PET Glitter (Crystal Clear 0.016" 45.016E, einseitig beschichtet zur Vermeidung der Bindung von Perlglanzpigmenten an die beschichtete Seite, Fa. RJA plastics GmbH) wurde mit 5 Gew.-% Luxan C001 ($D_{50}$ = 30 - 35 $\mu$m), Fa. Eckart, vermischt und homogenisiert. Anschließend wurden 10 Gew.-% einer 1:1 Mischung aus Laropal A81, Fa. BASF SE, Ludwigshafen, Deutschland, und Isopropanol zugegeben und erneut homogenisiert. Diese Mischung wurde in einer Schichtdicke von 3 mm auf ein Blech gestreut und anschließend im Trockenschrank bei 60°C für 15 min getrocknet. Durch die leichte Flüchtigkeit des Isopropanols liegt der Feststoffgehalt der Mischung nach Trocknung bei 100 Gew.-%.

**II Charakterisierung der erfindungsgemäßen Kompositpartikel**

**IIa Partikelgrößenmessung**

Die Größenverteilungskurve der Trägermaterialien und Perlglanzpigmente wurde mit einem Gerät der Fa. Malvern Instruments GmbH, 71083 Herrenberg (Gerät: Mastersizer 2000) gemäß Herstellerangaben bestimmt. Hierzu wurden ca. 0,1 g des entsprechenden Trägermaterials oder Perlglanzpigmentes als wässrige Suspension, ohne Zusatz von Dispergierhilfsmitteln, unter ständigem Rühren mittels einer Pasteurpipette in die Probenvorbereitungszelle des Messgerätes gegeben und dreifach vermessen. Aus den einzelnen Messergebnissen wurden die resultierenden Mittelwerte gebildet. Die Auswertung der Streulichtsignale erfolgte dabei nach der Mie-Theorie, welche auch Brechungs- und Absorptionsverhalten der Trägermaterialien bzw. der Perlglanzpigmente beinhaltet.

Unter der mittleren Größe $D_{50}$ wird im Rahmen dieser Erfindung der $D_{50}$-Wert der Summenhäufigkeitsverteilung der volumengemittelten Größenverteilungsfunktion, wie sie durch Laserbeugungsmethoden erhalten werden, verstanden. Der $D_{50}$-Wert gibt an, dass 50 % der Trägermaterialien bzw. Perlglanzpigmente einen Durchmesser aufweisen, der gleich oder kleiner als der angegebene Wert, beispielsweise 20 $\mu$m, ist. Entsprechend gibt der $D_{10}$- bzw. $D_{90}$-Wert an, dass 10 % bzw. 90 % der Trägermaterialien bzw. Perlglanzpigmente einen Durchmesser aufweisen, der gleich oder kleiner als der angegebene Wert ist.

**IIb Bestimmung der mittleren Dicke**

Die mittlere Dicke wurde anhand von Querschliffen der erfindungsgemäßen Kompositpartikel mit dem Rasterelektronenmikroskop Supra 35 (Fa. Carl Zeiss AG, 73447 Oberkochen, Deutschland) bestimmt. Hierbei wurden jeweils mindestens 100 Teilchen vermessen, um eine aussagefähige Statistik zu erhalten.

**IIc Rasterelektronenmikroskopische Aufnahmen**

Die rasterelektronenmikroskopischen Aufnahmen wurden anhand von Querschliffen der erfindungsgemäßen Kompositpartikel mit dem Rasterelektronenmikroskop Supra 35 (Fa. Carl Zeiss AG) erhalten.

**IId Lichtmikroskopische Aufnahmen**

Lichtmikroskopische Aufnahmen wurden anhand von Schwarz-Weiß Deckungskarten (Leneta Form 605 C, Fa. Leneta Company, Inc., Mahwah, New Jersey, USA) mit dem Mikroskop Axioskop 50 (Fa. Carl Zeiss AG) erstellt. Hierzu wurden die Partikel der jeweiligen Beispiele oder Vergleichsbeispiele auf die Schwarz-Weiß Deckungskarten aufgestreut.
Vor Aufstreuung der Kompositpartikel der erfindungsgemäßen Beispiele und der Partikel gemäß den Vergleichsbeispielen wurden die Schwarz-Weiß-Deckungskarten mittels Siebdruckverfahren (Sieb PET 1500 30-120 (Fäden pro cm), Fa. Sefar (9410 Heiden, Schweiz) mit dem lösemittelbasierenden Siebdruckbindemittel Libraspeed LIS (Fa. Marabu GmbH & Co. KG, 71732 Tamm, Deutschland) bedruckt. Für bessere Verdruckbarkeit und Untergrundbenetzung wurde das Siebdruckbindemittel mit 0,25 Gew.-% Byk 019 und 0,25 Gew.-% Dynwet 800 (beide Fa. BYK-Chemie GmbH, 46483 Wesel, Deutschland), jeweils bezogen auf das Gesamtgewicht der Siebdruckformulierung, versehen. Anschließend wurden die Partikel der Beispiele oder Vergleichsbeispiele unter einem Winkel von 45° das Siebdruckbindemittel aufgestreut. Überschüssiges Effektpigment wurde abgeklopft und anschließend 5 min bei 80°C im Trockenschrank getrocknet.

**IIe Glanzmessungen**

Die Glanzmessungen wurden anhand von Schwarz-Weiß-Deckungskarten (Leneta Form 605 C, Fa. Leneta) mit dem Gerät micro-TRI-gloss $\mu$ (Fa. Byk-Gardner GmbH, 82538 Geretsried, Deutschland) gemäß Herstellerangaben bei einer Messgeometrie von 20°, 60° und 85°, bezogen auf die Vertikale, jeweils auf weißem und schwarzem Untergrund durchgeführt. Eine Messgeometrie von 60° ist für den sogenannten "Mittelglanz" im Bereich von 10 bis 70 Glanzpunkten geeignet, wobei ein höherer Zahlenwert bei den Glanzpunkten einem höheren Glanz entspricht. Eine Messgeometrie von 20° ist für den sogenannten "Hochglanz" geeignet, d. h. liegt der Glanzwert bei der Messgeometrie von 60° bei über 70 Glanzpunkten, so wird bei einer Messgeometrie von 20° gemessen. Eine Messgeometrie von 85° ist für den sogenannten "Mattglanz" geeignet, d.h. liegt der Glanzwert bei der Messgeometrie von 60° bei weniger als 10 Glanzpunkten, so wird bei einer Messgeometrie von 85° gemessen. (Byk-Gardner, Katalog "Qualitätskontrolle für Lacke und Kunststoffe" 2011/2012, S. 16). Die nachstehend in Tabelle aufgeführten Glanzwerte stellen Mittelwerte aus jeweils fünf Einzelmessungen dar.
Hierzu wurde die Partikel der Beispiele oder Vergleichsbeispiele entweder auf die Schwarz-Weiß Deckungskarten, wie in Abschnitt IId beschrieben, aufgestreut oder die Schwarz-Weiß-Deckungskarten wurden hiermit im Siebdruckverfahren bedruckt. Für den Siebdruck wurde eine Siebdruckfarbe bestehend aus 15 Gew.-% XY aus den Beispielen und Vergleichsbeispielen und 85 Gew.-% Plastisol PH1046 (Fa. Pharetra Gesellschaft für textile Kunststoffanwendung mbH & Co. KG, 95152 Selbitz, Deutschland) hergestellt. Diese Siebdruckfarbe wurde in Abhängigkeit von der Partikelgröße mittels Siebdruckverfahren (Sieb PET 1500 8 - 300 (Fäden pro cm/Fadendicke in $\mu$m) bzw. 21 - 140 (Fäden pro cm/Fadendicke in $\mu$m), Fa. Sefar) auf Schwarz-Weiß-Deckungskarten gedruckt und anschließend bei 170° C im Trockenschrank 1 Minute getrocknet. Bei einer Partikelgröße von größer als 200 $\mu$m wurde das Sieb PET 1500 8 - 300, bei einer Partikelgröße bis zu 200 $\mu$m wurde das Sieb PET 1500 21 - 140 eingesetzt.

Tabelle 2: Glanzwerte der Partikel der Beispiele oder Vergleichsbeispiele anhand der Schwarz-Weiß-Deckungskarten aus IId

| Beispiel/Vergleichsbeispiel | Glanz 20°, weiß° | Glanz 20°, schwarz | Glanz 60°, weiß | Glanz 60°, schwarz | Glanz 85°, weiß | Glanz 85°, schwarz |
|---|---|---|---|---|---|---|
| Beispiel 1 | 3,0 | 2,0 | 3,2 | 2,2 | 0,5 | 0,5 |
| Beispiel 2 | 4,6 | 4,8 | 6,4 | 5,0 | 0,6 | 0,5 |
| Beispiel 3 | 8,8 | 7,6 | 16,2 | 14,6 | 2 | 2 |
| Vergleichsbeispiel 4 | 6,0 | 4,0 | 5,4 | 3,4 | 0,5 | 0,5 |
| Vergleichsbeispiel 5 | 8,4 | 6,2 | 8,2 | 6,0 | 0,6 | 0,6 |
| Vergleichsbeispiel 6 | 9,8 | 8,2 | 13,2 | 11,6 | 1,9 | 2,0 |
| Vergleichsbeispiel 7 | - | 4,0 | 4,6 | 3,0 | 0,6 | 0,5 |

Tabelle 3: Glanzwerte der Partikel der Beispiele oder Vergleichsbeispiele, welche im Siebdruckverfahren auf Schwarz-Weiß-Deckungskarten gedruckt wurden.

| Beispiel/Vergleichsbeispiel | Glanz 20°, weiß° | Glanz 20°, schwarz | Glanz 60°, weiß | Glanz 60°, schwarz | Glanz 85°, weiß | Glanz 85°, schwarz |
|---|---|---|---|---|---|---|
| Beispiel 1 | 12,8 | 9,2 | 53,0 | 47,0 | 70,6 | 74,4 |
| Beispiel 2 | 7,8 | 6,2 | 44,0 | 40,4 | 71,8 | 71,8 |
| Beispiel 3 | 7,8 | 6,0 | 41,8 | 39,0 | 51,8 | 57,2 |
| Vergleichsbeispiel 1 | 11,8 | 15,0 | 48,8 | 56,6 | 56,4 | 69,4 |
| Vergleichsbeispiel 2 | 7,6 | 7,2 | 38,8 | 38,6 | 51,6 | 65,2 |
| Vergleichsbeispiel 4 | 11,4 | 9,4 | 46,2 | 44,4 | 59,0 | 60,8 |
| Vergleichsbeispiel 5 | 8,2 | 5,0 | 43,2 | 35,2 | 60,6 | 61,0 |
| Vergleichsbeispiel 6 | 5,4 | 3,6 | 24,8 | 25,4 | 30,6 | 39,4 |
| Vergleichsbeispiel 7 | 4,2 | 3,6 | 23,8 | 24,8 | 27,2 | 31,8 |

**IIf Bestimmung des Hell-/ Dunkelflops (Flopindex)**

Der Hell-/ Dunkelflop (Flopindex) wurde anhand der Schwarz-Weiß-Deckungskarten aus IIe, welche im Siebdruckverfahren mit Partikeln der Beispiele oder Vergleichsbeispiele bedruckt wurden, mit dem Gerät BYK-mac (Fa. Byk-Gardner) ermittelt. Der Flopindex ist nach Alman folgendermaßen definiert (S. Schellenberger, M. Entenmann, A. Hennemann, P. Thometzek, Farbe und Lack, 04/2007, S. 130):

$$\text{Flopindex} = 2{,}69 \cdot (L_{E1} - L_{E3})^{1,11} / L_{E2}^{0,86}$$

wobei $L_{E1}$ für die Helligkeit des glanznahen Messwinkels (E1 = 15° zum Glanzwinkel), $L_{E2}$ für die Helligkeit des Messwinkels zwischen glanznahem und glanzfernen Winkel (E2 = 45° zum Glanzwinkel) und $L_{E3}$ für die Helligkeit des glanzfernen Messwinkels (E3 = 110° zum Glanzwinkel) steht.
Die in unten stehender Tabelle aufgeführten Werte wurden auf dem weißen Untergrund der Schwarz-Weiß-Deckungskarte vermessen.

**IIg Körnigkeit**

Die Körnigkeit G wurden anhand der Schwarz-Weiß-Deckungskarten aus Abschnitt IIe, welche im Siebdruckverfahren mit den Partikeln der Beispiele bzw. Vergleichsbeispiele bedruckt wurden, mit dem Gerät BYK-mac (Fa. Byk-Gardner) bestimmt.

Zur Bewertung der Körnigkeit nimmt die hochauflösende CCD-Kamera ein Bild bei diffuser Beleuchtung auf, die durch zwei weiß beschichtete Halbkugeln erzeugt wird. Das Bild wird mit Hilfe des Histogrammes der Helligkeitsstufen analysiert, wobei die Homogenität der hellen und dunklen Flächen in einem Körnigkeitswert zusammengefasst wird. Je höher dieser Wert ist, desto körniger erscheint die mit Partikeln der Beispiele oder Vergleichsbeispiele bedruckte Schwarz-Weiß-Deckungskarte bei diffuser Beleuchtung. Je höher die Körnigkeit einer mit den Partikeln der Beispiele oder Vergleichsbeispiele bedruckten Schwarz-Weiß-Deckungskarte unter diffusem Licht ist, als desto glitzernder wird diese unter direkter Beleuchtung visuell wahrgenommen (Byk-Gardner, Qualitätskontrolle für Lacke und Kunststoffe, 2011/2012, S. 98).

Tabelle 4: Helligkeit L*, Flopindex und Körnigkeit G

| Beispiel/Vergleichsbeispiel | L*, 15° | L*, 25° | L*, 45° | L*, 75° | L*, 110° | Flopindex | G |
|---|---|---|---|---|---|---|---|
| Beispiel 1 | 100,7 | 95,1 | 92,4 | 91,8 | 89,1 | 0,8 | 3,3 |
| Beispiel 2 | 100,7 | 94,3 | 89,8 | 90,2 | 87,9 | 1,0 | 4,8 |
| Beispiel 3 | 101,9 | 94,2 | 90,3 | 89,2 | 87,2 | 1,1 | 3,4 |
| Vergleichsbeispiel 1 | 110,1 | 96,4 | 86,7 | 75,2 | 73,6 | 3,1 | 22,5 |
| Vergleichsbeispiel 2 | 104,2 | 90,8 | 72,9 | 65,7 | 59,4 | 4,6 | 23,6 |
| Vergleichsbeispiel 3 | 103,5 | 77,8 | 61,1 | 58,3 | 57,0 | 5,6 | 22,6 |

Je größer der Zahlenwert des Flopindexes ist, desto stärker kommt der Hell-/ Dunkelflop zum Ausdruck. Beim Vergleich der Beispiele 1 bis 3 ist zu erkennen, dass mit steigender Partikelgröße der Kompositpartikel der Wert des Flopindexes zunimmt. Gleiches gilt auch für die Vergleichsbeispiele 1 bis 3. Ein Vergleich der Beispiele 1 bis 3 mit den Vergleichsbeispielen 1 bis 3 zeigt, dass bei letzteren der Wert des Flopindexes deutlich höher ist. Auch bei einer visuellen Beurteilung der mit den Vergleichbeispielen 1 bis 3 bedruckten Schwarz-Weiß-Deckungskarten verlieren diese außerhalb des Glanzwinkels ihren Metallglanz und erscheinen einem Betrachter dunkel. Dieser optische Effekt ist bei den Beispielen 1 bis 3 deutlich weniger ausgeprägt, was sich durch die Transparenz der erfindungsgemäßen Kompositpartikel erklärt. Aufgrund dieser Transparenz scheint außerhalb des Glanzwinkels der weiße Untergrund der Schwarz-Weiß-Deckungskarte durch.

Je höher der Zahlenwert G der Körnigkeit ist, desto körniger erscheint unter diffusem Licht die mit dem jeweiligen Beispiel bzw. Vergleichsbeispiel bedruckte Schwarz-Weiß-Deckungskarte einem Betrachter. Bei einem Vergleich der Beispiele 1 bis 3 mit den Vergleichsbeispielen 1 bis 3 sind bei letzteren deutlich höhere Werte für die Körnigkeit G zu beobachten. Dies wird auch bei einer visuellen Beurteilung der jeweiligen Schwarz-Weiß-Deckungskarten sichtbar. Die Vergleichsbeispiele 1 bis 3 zeigen im Unterschied zu den Beispielen 1 bis 3 einen deutlich höheren, uneinheitlich wirkenden Glitzereffekt, während bei den Beispielen 1 bis 3 ein homogeneres Erscheinungsbild wahrgenommen wird.

**III Anwendungstechnische Beispiele**

Anwendungstechnisches Beispiel 1: Applikation von Beispiel 1 auf einer Tapete im Siebdruckverfahren

Für den Siebdruck wurde eine Siebdruckfarbe bestehend aus 15 Gew.-% der Kompositpartikel aus Beispiel 1 und 85 Gew.-% Plastisol PH1046 (Fa. Pharetra) hergestellt. Diese Siebdruckfarbe wurde mittels Siebdruckverfahren (Sieb PET 1500 18 - 180 (Fäden pro cm/Fadendicke in $\mu$m), Fa. Sefar) auf eine bereits bedruckte Tapete aufgedruckt und anschließend bei 200° C in einem Trockentunnel getrocknet.

Anwendungstechnisches Beispiel 2: Applikation von Beispiel 2 auf einer Tapete im Aufstreuverfahren

Zur Veredelung einer mittels Tiefdruckverfahren bereits bedruckten Tapete wurde diese mit einer klebenden Schicht aus Optifoam, Fa. Veika, im Tiefdruckverfahren bedruckt. Anschließend wurden die Kompositpartikel aus Beispiel 2 auf diese Tapete mittels einer Aufstreueinheit appliziert. Nach Trocknung der Tapete im Trockentunnel bei 150° C wurden überschüssige Kompositpartikel abgesaugt.

Anwendungstechnisches Beispiel 3: Applikation von Beispiel 1 auf einer Tapete im wässrigen Tiefdruckverfahren

Für den Tiefdruck wurde eine Tiefdruckfarbe bestehend aus 15 Gew.-% der Kompositpartikel aus Beispiel 1 und 85 Gew.-% Rotostar Aqua 400 255 Medium, Fa. Eckart, hergestellt und mit Wasser auf eine Druckviskosität von 30 s im DIN Auslaufbecher Nr. 4, Fa. Byk-Gardner, eingestellt. Diese Tiefdruckfarbe wurde mittels Tiefdruckverfahren auf eine bereits bedruckte Tapete aufgedruckt und anschließend bei 150° C in einem Trockentunnel getrocknet.

Anwendungstechnisches Beispiel 4: Nagellack

| INCI Name | Produktname | Gew.-% | Hersteller |
|---|---|---|---|
| | Beispiel 1 | 3 | |
| Butylacetate, Ethylacetate, Nitrocellulose, Isopropyl Alcohol | International Lacquers Base 12616 | 98,00 | Fa. International Lacquers |

Anwendungstechnisches Beispiel 5: Nagellack

| INCI Name | Produktname | Gew.-% | Hersteller |
|---|---|---|---|
| | Beispiel 3 | 4 | |
| Butylacetate, Ethylacetate, Nitrocellulose, Isopropyl Alcohol | International Lacquers Base 12616 | 96,00 | Fa. International Lacquers |

Anwendungstechnisches Beispiel 6: Nagellack

| INCI Name | Produktname | Gew.-% | Hersteller |
|---|---|---|---|
| | Beispiel 2 | 2 | |
| | Syncrystal Silver | 2 | Fa. Eckart |
| Butylacetate, Ethylacetate, Nitrocellulose, Isopropyl Alcohol | International Lacquers Base 12616 | 96,00 | Fa. International Lacquers |

**Patentansprüche**

1. Kompositpartikel, umfassend ein Trägermaterial sowie wenigstens ein Perlglanzpigment,
   **dadurch gekennzeichnet,**
   **dass** das Trägermaterial und das wenigstens eine Perlglanzpigment ohne Haftmittel aneinander gebunden vorliegen, wobei das Perlglanzpigment auf der Oberfläche des Trägermaterials liegt und von dem Trägermaterial nicht umhüllt oder eingekapselt ist.

2. Kompositpartikel nach Anspruch 1,
   **dadurch gekennzeichnet,**
   **dass** das Trägermaterial eine Glasübergangstemperatur aufweist.

3. Kompositpartikel nach einem der vorstehenden Ansprüche,
   **dadurch gekennzeichnet,**
   **dass** das Trägermaterial plättchenförmig ist.

4. Kompositpartikel nach einem der vorstehenden Ansprüche 1 oder 2,
   **dadurch gekennzeichnet,**
   **dass** das Trägermaterial annähernd sphärisch, linsenförmig oder irregulär geformt ist.

5. Kompositpartikel nach einem der vorstehenden Ansprüche,
   **dadurch gekennzeichnet,**
   **dass** das Trägermaterial eine polygonale Form aufweist.

6. Kompositpartikel nach einem der vorstehenden Ansprüche,
   **dadurch gekennzeichnet,**
   **dass** das wenigstens eine Perlglanzpigment in weitgehend flächigem Kontakt mit dem Trägermaterial verbunden ist.

**7.** Kompositpartikel nach einem der vorstehenden Ansprüche,
**dadurch gekennzeichnet,**
**dass** das wenigstens eine Perlglanzpigment und das Trägermaterial stoffschlüssig miteinander verbunden sind.

**8.** Kompositpartikel nach einem der vorstehenden Ansprüche,
**dadurch gekennzeichnet,**
**dass** das Kompositpartikel ein erstes Trägermaterial umfasst, wobei das erste Trägermaterial an ein zweites Trägermaterial ohne Haftmittel gebunden ist, wobei das zweite Trägermaterial und das erste Trägermaterial des Kompositpartikels voneinander verschieden sind.

**9.** Kompositpartikel nach einem der vorstehenden Ansprüche,
**dadurch gekennzeichnet,**
**dass** das Trägermaterial ein Kunststoff ist.

**10.** Kompositpartikel nach Anspruch 9,
**dadurch gekennzeichnet,**
**dass** der Kunststoff ein thermoplastischer Kunststoff oder Elastomer ist.

**11.** Verfahren zur Herstellung eines Kompositpartikels nach einem der vorstehenden Ansprüche,
**dadurch gekennzeichnet,**
**dass** das Verfahren folgenden Schritt umfasst: Mischen von Trägermaterial und wenigstens einem Perlglanzpigment, wobei das Trägermaterial eine wenigstens teilweise erweichte Oberfläche aufweist.

**12.** Verwendung eines Kompositpartikels nach einem der Ansprüche 1 bis 9 zur Beschichtung von Substraten, vorzugsweise Tapeten und Druckerzeugnissen.

**13.** Verwendung eines Kompositpartikels nach einem der Ansprüche 1 bis 9 in einem Beschichtungsmaterial oder als Beschichtungsmaterial.

**14.** Beschichtungsmaterial,
**dadurch gekennzeichnet,**
**dass** es Kompositpartikel nach einem der Ansprüche 1 bis 9 enthält.

**15.** Beschichtungsmaterial nach Anspruch 13,
**dadurch gekennzeichnet,**
**dass** das Beschichtungsmaterial eine Farbe, Druckfarbe, Lack oder Kosmetikum ist.

**Claims**

**1.** Composite particle comprising a carrier material and at least one pearlescent pigment,
**characterised in that**
the carrier material and the at least one pearlescent pigment are present bonded to one another without adhesive, and the pearlescent pigment lies on the surface of the carrier material and is not enveloped or encapsulated by the carrier material.

**2.** Composite particle as claimed in claim 1,
**characterised in that**
the carrier material has a glass transition temperature.

**3.** Composite particle as claimed in one of the preceding claims, **characterised in that**
the carrier material is platelet-shaped.

**4.** Composite particle as claimed in one of preceding claims 1 or 2, **characterised in that**
the carrier material is approximately spherical, lens-shaped or of an irregular shape.

**5.** Composite particle as claimed in one of the preceding claims, **characterised in that**
the carrier material has a polygonal shape.

**6.** Composite particle as claimed in one of the preceding claims, **characterised in that** the at least one pearlescent pigment is joined to the carrier material with a largely flat contact.

**7.** Composite particle as claimed in one of the preceding claims, **characterised in that** the at least one pearlescent pigment and the carrier material are joined to one another by a material bond.

**8.** Composite particle as claimed in one of the preceding claims, **characterised in that** the composite particle comprises a first carrier material, which first carrier material is bonded to a second carrier material without adhesive, and the second carrier material and the first carrier material of the composite particle are different from one another.

**9.** Composite particle as claimed in one of the preceding claims, **characterised in that** the carrier material is a plastic.

**10.** Composite particle as claimed in claim 9, **characterised in that** the plastic is a thermoplastic or thermoplastic or elastomer.

**11.** Method of producing a composite particle as claimed in one of the preceding claims, **characterised in that** the method comprises the following steps: mixing carrier material and at least one pearlescent pigment, which carrier material has an at least partially softened surface.

**12.** Use of a composite particle as claimed in one of claims 1 to 9 for coating substrates, preferably wallpaper and printed products.

**13.** Use of a composite particle as claimed in one of claims 1 to 9 in a coating material or as a coating material.

**14.** Coating material, **characterised in that** it contains composite particles as claimed in one of claims 1 to 9.

**15.** Coating material as claimed in claim 13, **characterised in that** the coating material is a paint, printing ink, varnish or cosmetic.

**Revendications**

**1.** Particule composite, comprenant un matériau support ainsi qu'au moins un pigment nacré, **caractérisée en ce que** le matériau support et le ou les pigments nacrés se présentent liés l'un à l'autre sans adhésif, le pigment nacré se trouvant sur la surface du matériau support et n'étant pas enveloppé ou encapsulé par le matériau support.

**2.** Particule composite selon la revendication 1, **caractérisée en ce que** le matériau support présente une température de transition vitreuse.

**3.** Particule composite selon l'une des revendications précédentes, **caractérisée en ce que** le matériau support est lamellaire.

**4.** Particule composite selon l'une des revendications précédentes 1 ou 2, **caractérisée en ce que** le matériau support a une forme approximativement sphérique, lenticulaire ou irrégulière.

**5.** Particule composite selon l'une des revendications précédentes, **caractérisée en ce que** le matériau support présente une forme polygonale.

**6.** Particule composite selon l'une des revendications précédentes, **caractérisée en ce que** le ou les pigments nacrés sont liés au matériau support selon un contact essentiellement en pleine surface.

**7.** Particule composite selon l'une des revendications précédentes, **caractérisée en ce que** le ou les pigments nacrés et le matériau support sont liés l'un à l'autre par liaison de matière.

**8.** Particule composite selon l'une des revendications précédentes, **caractérisée en ce que** la particule composite comprend un premier matériau support, le premier matériau support étant lié à un deuxième matériau support sans adhésif, le deuxième matériau support et le premier matériau support de la particule composite étant différents l'un de l'autre.

**9.** Particule composite selon l'une des revendications précédentes, **caractérisée en ce que** le matériau support est un matériau plastique.

**10.** Particule composite selon la revendication 9, **caractérisée en ce que** le matériau plastique est un matériau thermoplastique ou un élastomère.

**11.** Procédé de fabrication d'une particule composite selon l'une des revendications précédentes, **caractérisé en ce que** le procédé comprend l'étape suivante : mélange du matériau support et d'au moins un pigment nacré, le matériau support présentant une surface au moins partiellement ramollie.

**12.** Utilisation d'une particule composite selon l'une des revendications 1 à 9 pour revêtir des substrats, de préférence des papiers-peints et des produits imprimés.

**13.** Utilisation d'une particule composite selon l'une des revendications 1 à 9 dans un matériau de revêtement ou en tant que matériau de revêtement.

**14.** Matériau de revêtement, **caractérisé en ce qu'**il contient des particules composites selon l'une des revendications 1 à 9.

**15.** Matériau de revêtement selon la revendication 13, **caractérisé en ce que** le matériau de revêtement est une peinture, une encre d'imprimerie, un vernis ou un produit cosmétique.

Fig. 1

Fig. 2

Fig. 3

Fig. 4

Fig. 5

Fig. 6

Fig. 7

200 µm

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008064201 A1
- EP 1520876 A1
- DE 19941607 A1
- DE 10313663 A1
- US 20110112234 A1

- EP 0289240 A1
- WO 2004056716 A1
- WO 2005063637 A1
- EP 1980594 B1
- WO 2009103322 A1

**In der Beschreibung aufgeführte Nicht-Patentliteratur**

- **BYK-GARDNER.** *Qualitätskontrolle für Lacke und Kunststoffe,* 2011, 61, , 62

- **BYK-GARDNER.** *Qualitätskontrolle für Lacke und Kunststoffe,* 2011, 98